# EUROPÄISCHE PATENTANMELDUNG

(11) **EP 3 906 896 A1**
(43) Veröffentlichungstag der Anmeldung: **10.11.2021**
(21) Anmeldenummer: 20173383.9
(22) Anmeldetag: 07.05.2020
(51) Int. Cl.: A61F 2/30, A61F 2/46, A61B 17/56, A61F 2/36, A61F 2/40

(54) **HERSTELLUNG VON SPACERN IN GIESSFORM MIT KNICK- ODER KLEMMEINRICHTUNG**

(71) Anmelder: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Schultheiss & Sterzel Patentanwälte PartG mbB

(57) **Zusammenfassung**

Die Erfindung betrifft eine Vorrichtung zum Herstellen eines Spacers aus Knochenzementteig aufweisend eine Gießform (1) mit einem Innenraum (3) zum Formen des Spacers mit einer Einfüllöffnung (2), einen Anschluss (4) zum flüssigkeitsdichten Anschließen einer Knochenzementkartusche (10), eine Leitung (5) zum Einleiten von Knochenzementteig, wobei die Leitung (5) den Anschluss (4) mit der Einfüllöffnung (2) für den Knochenzementteig durchlässig verbindet und die Leitung (5) mit der Einfüllöffnung (2) in den Innenraum (3) der Gießform (1) mündet, wobei die Leitung (5) einen verformbaren Bereich (6) aufweist, der örtlich neben der Einfüllöffnung (2) angeordnet ist, eine Sollknickstelle im verformbaren Bereich (6), wobei der innere Leitungsquerschnitt der Leitung (5) oder die Querschnittsfläche der Leitung (5) durch Knicken der Leitung (5) an der Sollknickstelle der Leitung (5) in dem verformbaren Bereich (6) um mindestens 90% reduzierbar ist, und/oder eine Klemmeinrichtung zum Klemmen der Leitung (5) im verformbaren Bereich (6), mit der der innere Leitungsquerschnitt der Leitung (5) oder die Querschnittsfläche der Leitung (5) in dem verformbaren Bereich (6) durch Klemmen der Leitung (5) in dem verformbaren Bereich (6) um mindestens 90% reduzierbar ist, so dass die Leitung (5) in dem so geklemmten Zustand in dem geklemmten Bereich für den Knochenzementteig undurchlässig ist.

Die Erfindung betrifft auch ein Verfahren zur Herstellung von Spacern.

## Beschreibung

Die Erfindung betrifft eine Vorrichtung zum Herstellen eines Spacers durch Aushärten von Knochenzementteig. Der Spacer ist als temporärer Platzhalter dazu vorgesehen, im medizinischen Anwendungsbereich ein Gelenk oder einen Teil eines Gelenks umfassend eine artikulierende Oberfläche des Gelenks temporär zu ersetzen. Bevorzugt ist der Spacer zum temporären Ersetzen eines Hüftgelenks oder eines Schultergelenks geeignet und vorgesehen. Dementsprechend ist die Vorrichtung bevorzugt zum Herstellen eines Hüftgelenkspacers oder eines Schultergelenkspacers vorgesehen. Die Erfindung betrifft auch ein Verfahren zur Herstellung eines solchen Spacers mit einer solchen Vorrichtung.

Die Vorrichtung ist insbesondere ein Kit zur intraoperativen Herstellung von einteiligen Hüft- und Schulterspacern, die als temporäre Platzhalter (Spacer) im Rahmen von zweizeitigen Revisionen von infizierten Hüft- und Schultertotalgelenkendoprothesen für die Interimsphase bestimmt sind. Der Kit ist für die Herstellung von Spacern mit niedrigviskosem und hochviskosem Polymethylmethacrylat-Knochenzementteig geeignet. Ein weiterer Gegenstand der Erfindung ist ein Verfahren zur Herstellung von Hüft- und Schulterspacern mit einem solchen Kit.

Gelenk-Endoprothesen, wie Hüftgelenk-Endoprothesen und Schultergelenk-Endoprothesen, werden in großem Umfang weltweit implantiert. Leider kommt es in einem geringen Prozentsatz vor, dass Gelenk-Endoprothesen von mikrobiellen Keimen, besonders Grampositiven Bakterien als auch Gram-negativen Bakterien und in sehr geringem Umfang von Hefen und Pilzen, besiedelt werden. Diese mikrobiellen Keime, hauptsächlich typische Hautkeime wie Staphylococcus aureus und Staphylococcus epidermidis, können während einer chirurgischen Operation (OP) in den Patienten gelangen. Daneben ist es auch möglich, dass mikrobielle Keime hämatogen Gelenk-Endoprothesen erreichen. Bei einer Besiedlung von Gelenk-Endoprothesen mit mikrobiellen Keimen wird das umliegende Knochen- und Weichgewebe mit infiziert und von den mikrobiellen Keimen geschädigt.

Stand der Technik sind vor allem zwei Behandlungsverfahren für infizierte Gelenk-Endoprothesen, die einzeitige septische Revision und die zweizeitige septische Revision. Bei der einzeitigen Revision wird innerhalb einer OP zuerst die infizierte Gelenk-Endoprothesen entfernt, anschließend radikal debridiert und dann wird eine Revisions-Gelenk-Endoprothese implantiert.

Bei zweizeitigen septischen Revisionen wird in einer ersten OP zuerst die infizierte Gelenk-Endoprothese entfernt, dann wird debridiert und anschließend erfolgt die Implantation eines Spacers. Ein Hüftgelenkspacer besteht aus einem Schaft, einem Kragen, einem Hals und einem Kugelkopf und ist den Hüftgelenk-Endoprothesen in Form und Größe nachgebildet. Analog ist ein Schultergelenkspacer einer Schultergelenk-Endoprothesen in Form und Größe nachgebildet. Der Spacer wird mit Knochenzement an dem jeweiligen Knochen verankert, also beispielsweise bei Hüftgelenkspacern am proximalen Femur beziehungsweise im Femurkanal verankert. Der Spacer verbleibt bis zu mehreren Wochen im Patienten bis die Entzündung abgeklungen ist und die klinischen Entzündungsmarker zurückgegangen sind. Dann wird in einer zweiten OP der Spacer entfernt und nach erneutem Debridement eine Revisions-Gelenk-Endoprothese implantiert.

Bei Spacern werden dem Zementpulver vor der eigentlichen Spacerherstellung Antibiotika zugesetzt. Mit diesem antibiotisch modifizierten Knochenzementpulver wird anschließend durch Beimischen von Monomerflüssigkeit ein Knochenzementteig hergestellt und aus diesem Knochenzementteig werden Spacer gegossen, die dann durch Polymerisation mit Hilfe der dem Zementpulver zugesetzten Monomerflüssigkeit aushärten. Der Knochenzementteig schließt dabei die Antibiotika im Wesentlichen ein. Die in den oberflächennahen Bereichen befindlichen Antibiotika-Partikel werden bei Einwirkung von Körperflüssigkeiten, wie Wundsekret, herausgelöst. Die Wirkstofffreisetzung ist initial am höchsten und nimmt dann im Verlauf von mehreren Tagen ab.

Die US 2010/0042213 A1 offenbart eine Hüftgelenkprothese mit einem Reservoir einer Flüssigkeit im Inneren des Implantats. Aus der WO 2017/178951 A1 ist ein Hüftspacer mit Vertiefungen bekannt, wobei in den Vertiefungen eine Substanz zur Behandlung des Knochens eingebracht werden kann. In dem Patent US 6 245 111 B1 wird eine Hüftgelenksprothese vorgeschlagen, deren Oberflächen mit einem Antibiotikum beschichtet sind. Das Patent US 5 681 289 offenbart eine Einrichtung zum Verteilen eines flüssigen Wirkstoffs mit Hilfe einer Blase im Inneren der Einrichtung. Keine der genannten Prothesen ist zum Erzeugen eines Spülkreislaufs geeignet. In der EP 1 991 170 B1 und der US 2011/0015754 A1 werden ein Hüftgelenkspacer beschrieben, die Wirkstoffe enthalten. Die US 2019/0290833 A1 offenbart einen spülbaren Hüftgelenkspacer, mit dem ein Flüssigkeitskreislauf erzeugbar ist. In der WO 2016/205077 A1 und der US 8 900 322 B2 werden weitere Spacer mit einer Spülfunktion beschrieben.

Es ist bekannt, mit Antibiotika ausgerüstete Spacer zu verwenden. Spacer können einerseits vom OP-Personal während der OP selbst aus PMMA-Knochenzementpulver, Antibiotika und Monomerflüssigkeit hergestellt werden, zum Beispiel mit einer Spacerform, wie sie beispielsweise in den Patenten DE 10 2015 104 704 B4 oder EP 2 617 393 B1 beschrieben sind, und andererseits ist es auch üblich, industriell aus Knochenzement vorgefertigte Hüftgelenkspacer einzusetzen. Kunststoffgießformen zur intraoperativen Herstellung von einteiligen Hüftspacern werden in der US 6 361 731 B1 beschrieben. Diese Gießformen sind transparent und haben zwei separate Einfüllöffnungen. Dadurch kann auch hochviskoser Knochenzementteig mit geringem Druck in die Gießform eingebracht werden, weil die Fließwege des Knochenzementteigs relativ kurz sind. Bei der Verwendung von nicht hochviskosem Knochenzementteig besteht die Gefahr, dass Knochenzementteig nach erfolgter Füllung der Gießform vor der einsetzenden Aushärtung wieder aus den Einfüllöffnungen herausfließt.

In einer Weiterentwicklung wurden in den Patentschriften US 7 637 729 B2, US 7 789 646 B2, US 8 480 389 B2 und US 8 801 983 B2 mehrteilige Gießformen für die Herstellung modularer Hüftspacer vorgeschlagen. Diese modularen Hüftspacer bestehen aus einem Spacerkopf und einem separaten Schaft. Daher sind eine Gießform für den Spacerkopf und eine separate Gießform für den Schaft notwendig. Die Gießform für den Schaft ist einteilig und besitzt an der Einfüllöffnung ein Gewinde zur Verbindung der Gießform mit einer Zementkartusche enthaltend den Knochenzementteig.

In der US 2007/0222114 A1 wird eine Hüftspacerform beschrieben. Diese Spacerform besteht aus einer Vielzahl von Formsegmenten, die miteinander verbunden werden. Durch die Vielzahl der Segmente kann die Spacerform recht genau an die anatomischen Gegebenheiten des Patienten angepasst werden. Die Spacerform-Segmente werden mittels Schraubschellen aneinandergefügt. Durch Kanäle in der Spacerform wird ein PMMA-Knochenzementteig (Polymethylmethacrylat-Knochenzementteig) eingeführt. Durch den komplexen Aufbau der Gießform ist es sehr aufwändig, die Spacerform-Segmente zusammenzufügen und nach erfolgter Aushärtung des PMMA-Knochenzementteigs den Hüftspacer zu entnehmen.

In der WO 2009/073 781 A2 wird eine Spacerform für einen Hüftspacer vorgeschlagen, die aus zwei Teilen besteht, die in Bezug zueinander verschoben werden können, um die Länge des Schafts anpassen zu können. Eine weitere Gießform ist in der EP 2 522 310 A1 offenbart. Diese Vorrichtung besteht aus wenigstens zwei Teilen, wobei in einem ersten Teil eine Einschubabschnitt und im zweiten Teil eine Einschubaufnahme angeordnet sind. Beide Teile sind in einander steckbar und bilden eine Gießform für die Herstellung des Schafts des Hüftspacers. In der EP 2 787 928 A1 wird eine komplexe Gießform beschrieben. Diese ermöglicht die Herstellung von Hüftspacern mit unterschiedlichen Kugelköpfen. Die Fixierung der Elemente der Gießform erfolgt über Verbindungselemente. Im Patent US 7 789 646 B2 wird eine Gießform beschrieben, bei der mit einem Stöpsel die Einfüllöffnung der Gießform verschlossen werden kann, wenn der Knochenzementteig in die Gießform eingebracht wurde. Zuvor muss jedoch die Gießform von der Knochenzementkartusche abgeschraubt werden. Bei Verwendung von nicht hochviskosem Knochenzementteig ist es daher möglich, wenn die Gießform ungünstig gehalten wird, dass Knochenzementteig während der Trennung der Gießform von der Knochenzementkartusche ausläuft, bevor der Stöpsel eingeschraubt beziehungsweise eingesteckt ist.

Die Aufgabe der vorliegenden Erfindung besteht also darin, die Nachteile des Stands der Technik zu überwinden. Insbesondere besteht die Aufgabe der Erfindung in der Entwicklung einer kostengünstigen und leicht anwendbaren Vorrichtung zum Herstellen eines Spacers durch Aushärten von Knochenzementteig und in der Entwicklung eines einfach und kostengünstig durchführbaren Verfahrens zum Herstellen eines Spacers durch Aushärten von Knochenzementteig, mit dem vom medizinischen Personal im OP unter Verwendung von Knochenzementteig, insbesondere von Polymethylmethacrylat-Knochenzement, einteilige Spacer, insbesondere Hüft- und Schulterspacer, hergestellt werden können. Hüft- und Schulterspacer sind ähnlich aufgebaut. Sie bestehen aus einem Schaft und einem Kopf (Spacerkopf). Ein Metallkern kann zur mechanischen Stabilisierung im Inneren des Hüft- und Schulterspacer angeordnet sein beziehungsweise werden. Es soll möglich sein, sowohl mit (Polymethylmethacrylat-)Knochenzementteig mit niedriger beziehungsweise mit nicht hoher Viskosität als auch mit hoher Viskosität Spacer herzustellen. Die Vorrichtung soll einfach und für Fehler unanfällig anwendbar sein und das Verfahren einfach und sicher umsetzbar sein. Für die vollständige Füllung einer Gießform mit einem hochviskosem Knochenzementteig ist ein hoher Einpressdruck erforderlich. Die Gießform der Vorrichtung soll dem hohen Einpressdruck widerstehen. Die Gießform soll daher möglichst einem Druck von 10 N/cm² standhalten.

Die Vorrichtung soll so beschaffen sein, dass Knochenzementteig beziehungsweise fluider Knochenzementteig aus einer Knochenzementkartusche in eine Gießform eingepresst werden kann, um den Spacer innerhalb der Gießform durch Aushärten des Knochenzementteigs in der Gießform zu formen. Bei Verwendung von nicht hochviskosem Knochenzementteig soll nach Füllung der Gießform der Knochenzementteig nicht aus der Gießform ausfließen, damit der Knochenzementteig sich an die Innenwände der Gießform andrücken kann, um ebenmäßige Oberflächen des Spacers zu erzeugen. Dazu ist es notwendig, die Gießform so zu gestalten, dass ein Auslaufen von nicht hochviskosem Knochenzementteig aus der Gießform während der Trennung der Gießform von der Knochenzementkartusche sicher verhindert wird. Dieses Verschließen soll möglich sein, ohne dass in der Wandung der Gießform Öffnungen oder Ventile innerhalb der Gießform oder der Leitungen für den Knochenzementteig notwendig sind. Öffnungen in der Wandung der Gießform können zur Undichtigkeit der Gießform führen, wenn der Knochenzementteig unter hohem Druck in die Gießform eingepresst wird. Weiterhin soll der Angussbereich der Gießform so gestaltet sein, dass einerseits eine leichte Befüllung der Gießform mit Knochenzementteig möglich ist und dass andererseits nach erfolgter Aushärtung des Knochenzementteigs die Angussreste leicht entfernt werden können oder zumindest ein verbleibender Anguss die Funktion des Spacers nicht stört.

Die Aufgaben der Erfindung werden gelöst durch eine Vorrichtung zum Herstellen eines Spacers durch Aushärten von Knochenzementteig, wobei der Spacer dazu vorgesehen ist, im medizinischen Bereich ein Gelenk oder einen Teil eines Gelenks umfassend eine artikulierende Oberfläche des Gelenks temporär zu ersetzen, insbesondere ein Hüftgelenk oder ein Schultergelenk temporär zu ersetzen, die Vorrichtung aufweisend
eine Gießform mit einem Innenraum zum Formen des Spacers aus Knochenzementteig mit einer Einfüllöffnung zum Einfüllen eines Knochenzementteigs,
einen Anschluss zum flüssigkeitsdichten Anschließen einer Knochenzementkartusche,
eine Leitung zum Einleiten von Knochenzementteig, wobei die Leitung den Anschluss mit der Einfüllöffnung für den Knochenzementteig durchlässig verbindet und die Leitung mit der Einfüllöffnung in den Innenraum der Gießform mündet, wobei die Leitung einen verformbaren Bereich aufweist, der örtlich neben der Einfüllöffnung angeordnet ist,
eine Sollknickstelle im verformbaren Bereich der Leitung, wobei der innere Leitungsquerschnitt der Leitung oder die Querschnittsfläche der Leitung durch Knicken der Leitung an der Sollknickstelle der Leitung in dem verformbaren Bereich um mindestens 90% reduzierbar ist, so dass die Leitung in dem so entstandenen Knick für den Knochenzementteig undurchlässig ist, und/oder
eine Klemmeinrichtung zum Klemmen der Leitung im verformbaren Bereich, mit der der innere Leitungsquerschnitt der Leitung oder die Querschnittsfläche der Leitung in dem verformbaren Bereich durch Klemmen der Leitung in dem verformbaren Bereich um mindestens 90% reduzierbar ist, so dass die Leitung in dem so geklemmten Zustand in dem geklemmten Bereich für den Knochenzementteig undurchlässig ist.

Unter dem inneren Leitungsquerschnitt ist der minimale freie Leitungsquerschnitt der Leitung zu verstehen, der zur Leitung des Knochenzementteigs vorgesehen ist. Bei einem elliptischen Leitungsquerschnitt wäre dies der kurze Nebenachse und nicht die längere Hauptachse. Es reicht also aus, wenn der innere Leitungsquerschnitt sehr schmal wird. Das ist ausreichend, weil Knochenzementteige üblicherweise eher hochviskos sind und daher ein nennenswertes Fließen des Knochenzementteigs durch zu schmale Kanäle nicht möglich ist.

Die Querschnittsfläche der Leitung ist die offene Fläche senkrecht zur Fließrichtung in der Leitung, die zum Leiten des Knochenzementteigs in der Leitung zur Verfügung steht.

Unter einem Knochenzementteig beziehungsweise einem fluiden Knochenzementteig ist ein gemischter (also zum Einsatz bereiter) Knochenzementteig zu verstehen, der eine zähflüssige Konsistenz hat. Die Viskosität eines Knochenzementteigs entspricht vorzugsweise der von Honig oder hat eine noch zähflüssigere Konsistenz, das heißt höhere Viskosität. Die Begriffe fluider Knochenzement und Knochenzementteig werden synonym verwendet.

Der Knochenzementteig ist vorzugsweise ein PMMA-Knochenzementteig.

Es kann auch vorgesehen sein, dass die Klemmeinrichtung auch zum Knicken der Leitung in dem verformbaren Bereich vorgesehen und geeignet ist. Hierdurch wird eine weitere Möglichkeit zum Abdichten des Innenraums der Gießform mit der Vorrichtung ermöglicht.

Es kann auch vorgesehen sein, dass die Vorrichtung eine Knickeinrichtung zum manuellen Knicken der Leitung in der Sollknickstelle mit der Knickeinrichtung aufweist, so dass der innere Leitungsquerschnitt der Leitung durch das Knicken der Leitung an der Sollknickstelle der Leitung mit der Knickeinrichtung in dem verformbaren Bereich um mindestens 90% reduzierbar ist, so dass die Leitung in dem so geknickten Zustand in dem geknickten Bereich für den Knochenzementteig undurchlässig ist. Alternativ oder zusätzlich kann vorgesehen sein, dass die Sollknickstelle durch manuelles Knicken der Leitung an der Sollknickstelle der Leitung in dem verformbaren Bereich um mindestens 90% reduzierbar ist, so dass die Leitung in dem so geknickten Zustand in dem geknickten Bereich für den Knochenzementteig undurchlässig ist.

Flüssigkeitsdicht bedeutet, dass der nicht ausgehärtete also fluide Knochenzementteig und bevorzugt auch eine flüssige Monomerflüssigkeit als Ausgangskomponente des Knochenzements nicht ausfließen können, also beispielsweise nicht durch die Verbindung am Anschluss ausfließen oder hindurchdringen können.

Es kann bevorzugt auch vorgesehen sein, dass der Innenraum eine Negativform eines Gelenkkopfs und eines Schafts des Spacers bildet, insbesondere eines Hüftgelenkkopfs oder Schultergelenkkopfs bildet.

Es kann ferner vorgesehen sein, dass die Gießform zweiteilig oder mehrteilig ist, wobei vorzugsweise die Teile der Gießform über Flansche flüssigkeitsdicht aneinander befestigbar sind. Besonders bevorzugt ist die Gießform zweiteilig.

Die Gießform soll einem Druck von 10 N/cm² standhalten, um auch hochviskosen Knochenzementteig verwenden zu können.

In der vorliegenden Patentanmeldung werden die Richtungsbezeichnungen "proximal", "distal" und "lateral" sowie die Ebenen-bezeichnungen "Sagittalebene", "Frontalebene" und "Transversalebene" in Bezug auf den Spacer oder die Gießform so verwendet, wie dies bei dem im Patienten eingesetzten Zustand als anatomische Hauptrichtung oder als Körperebene zu verstehen wäre. Dabei bedeutet "proximal" zum Körperzentrum hin und "distal" vom Körperzentrum entfernt.

Der Schaft ist zur Verbindung mit einem Knochen (im Fall von Hüftgelenkspacern mit dem Femur) vorgesehen und ist vorzugsweise zu diesem Zweck in ein proximales Ende des präparierten Knochens beziehungsweise in den Knochenkanal einführbar.

Der verformbare Bereich soll vorzugsweise derart verformbar sein, dass die Leitung durch eine Verformung für den Knochenzementteig dicht verschließbar ist, wobei die Wandung dabei für den Knochenzementteig undurchlässig bleibt, also kein Knochenzementteig durch die Wandung nach außen fließen kann.

Durch das Klemmen oder Knicken soll die Leitung also vorzugsweise die Gießform nach außen flüssigkeitsdicht abdichten.

Bevorzugt kann vorgesehen sein, dass die Vorrichtung zur Herstellung eines Spacers, insbesondere eines Hüftgelenkspacers oder Schultergelenkspacers, zur Anwendung zumindest eines antibiotischen und/oder antimykotischen Wirkstoffs geeignet ist.

Bevorzugt ist der Spacer einteilig aus einem biokompatiblen Knochenzementteig, wie Polymethylmethacrylat (PMMA), zu fertigen, wobei besonders bevorzugt das PMMA wenigsten ein aus dem PMMA lösbares Antibiotikum und/oder Antimykotikum enthält.

Bevorzugt werden beim Klemmen oder Knicken Wandungen der Leitung im verformbaren Bereich aneinander gedrückt und dadurch die Leitung verschlossen. Hierdurch kann eine auch bei unter Druck stehendem Knochenzementteig eine Abdichtung der Leitung erreicht werden.

Unter einem Knicken der Leitung wird ein Knicken der Leitung senkrecht zur Achse beziehungsweise Flussrichtung der Leitung verstanden, so dass ein Knickwinkel in der Leitung entsteht.

Unter einer Sollknickstelle ist eine strukturelle mechanische Schwachstelle in der Leitung beziehungsweise im verformbaren Bereich der Leitung zu verstehen, an der die Leitung bei einem Knicken der Anschlusses gegen einen dem Anschluss gegenüberliegende Seite eines den Innenraum der Gießform begrenzenden Teil der Gießform senkrecht zur Leitungsachse knicken wird. Bei einem Knicken des Anschlusses gegen die Gießform (einer Drehung senkrecht zur Leitungsachse der Leitung) wird die Leitung also an dieser Sollknickstelle zuerst nachgeben und sich plastisch verformen und dabei die Leitung für den Knochenzementteig undurchlässig verschließen.

Bei erfindungsgemäßen Vorrichtungen kann vorgesehen sein, dass der Innenraum der Gießform durch das Knicken oder das Klemmen für den Knochenzementteig nach außen verschließbar ist, so dass der Knochenzementteig nicht aus der Gießform herausfließen kann, wenn die Leitung in dem so geknickten oder in dem so geklemmten Zustand ist.

Hierdurch wird sichergestellt, dass der Knochenzementteig nach dem Klemmen und/oder Knicken der in dem Innenraum der Gießform verbleibt, um den Spacer zu formen.

Des Weiteren kann vorgesehen sein, dass der verformbare Bereich angrenzend zur Einfüllöffnung angeordnet ist oder der verformbare Bereich der Leitung in einem Abstand von nicht mehr als 10 mm zur Einfüllöffnung beginnt, bevorzugt in einem Abstand von nicht mehr als 2 mm zur Einfüllöffnung beginnt.

Hiermit wird verhindert, dass ein ungewünschter größerer Anguss entsteht, der nach dem Aushärten des Knochenzementteigs und Entnahme des Spacers aus der Gießform aufwendig entfernt werden müsste.

Ferner kann vorgesehen sein, dass die Leitung in dem verformbaren Bereich durch manuelle Krafteinwirkung beim Knicken oder beim Klemmen mit der Klemmeinrichtung derart verformbar ist, dass der innere Leitungsquerschnitt der Leitung oder die Querschnittsfläche der Leitung durch Knicken oder Klemmen der Leitung an der Sollknickstelle der Leitung in dem verformbaren Bereich um mindestens 95% reduzierbar ist, bevorzugt um mindestens 99% reduzierbar ist.

Hierdurch können auch dünnflüssigere Knochenzementteige mit einer weniger viskosen Konsistenz als hochviskose Knochenzementteige in dem Innenraum der Gießform gehalten werden.

Es kann auch vorgesehen sein, dass die Leitung zumindest im verformbaren Bereich durch die Gießform gebildet ist, vorzugsweise die gesamte Leitung durch die Gießform gebildet ist.

Hierdurch wird keine separate Leitung benötigt, die mit der Gießform flüssigkeitsdicht verbunden werden müsste. Die Wandstärke der Gießform kann bei einer geeigneten Klemmeinrichtung überall gleich sein, das heißt auch in dem verformbaren Bereich der Leitung. Durch diese Maßnahme kann ein zusätzliches Bauteil der Vorrichtung eingespart werden.

Auch kann vorgesehen sein, dass der Anschluss an einer Seite der Gießform innerhalb der Gießform angeordnet und mit der Gießform verbunden ist, wobei der Anschluss von außerhalb der Gießform zugänglich ist, wobei bevorzugt der Anschluss flüssigkeitsdicht mit der Gießform verbunden ist und den einzigen Zugang für den Knochenzementteig in die Gießform bereitstellt.

Hierdurch kann der Anschluss direkt an der Gießform angeordnet sein, was die Handhabung vereinfacht und die gesamte Vorrichtung kompakter macht.

Bevorzugt kann ferner vorgesehen sein, dass die Vorrichtung eine Knochenzementkartusche zum Mischen von Knochenzement-Ausgangskomponenten und zum Austragen von gemischtem Knochenzementteig aus der Knochenzementkartusche heraus aufweist, bevorzugt eine Knochenzementkartusche zum Mischen von Polymethylmethacrylat-Knochenzement-Ausgangskomponenten und zum Austragen von gemischtem Polymethylmethacrylat-Knochenzementteig aus der Knochenzementkartusche heraus aufweist, wobei besonders bevorzugt die Knochenzementkartusche die Knochenzement-Ausgangskomponenten zur Herstellung des Knochenzements in voneinander getrennten Kompartimenten beinhaltet.

Hierdurch wird die Vorrichtung weiter komplettiert, da mit der Vorrichtung dann auch der Knochenzementteig bereitgestellt werden kann, der zum Füllen der Gießform und zum Herstellen des Spacers in der Gießform gebraucht wird. Die Vorrichtung beinhaltet dann alle wesentlichen Teile zum Herstellen des Spacers.

Dabei kann vorgesehen sein, dass die Vorrichtung ein Adapterelement aufweist, das mit der Knochenzementkartusche verbunden ist oder verbindbar ist, wobei das Adapterelement lösbar und formschlüssig mit dem Anschluss verbunden oder verbindbar ist, so dass ein Innenraum der Knochenzementkartusche über das Adapterelement für Knochenzementteig durchlässig mit der Leitung verbunden oder verbindbar ist, wobei vorzugsweise das Adapterelement mit dem Anschluss formschlüssig, lösbar und flüssigkeitsdicht verbindbar oder verbunden ist und/oder das Adapterelement einen Kartuschenkopf der Knochenzementkartusche bildet.

Hierdurch lässt sich die Knochenzementkartusche auf einfache Weise und für den Knochenzementteig dicht mit der Gießform verbinden.

Des Weiteren kann vorgesehen sein, dass die Gießform und/oder die Leitung aus einer Kunststofffolie besteht oder bestehen oder im Wesentlichen aus einer Kunststofffolie besteht oder bestehen oder die Gießform und/oder die Leitung aus zwei oder mehreren Kunststofffolien aufgebaut ist oder sind, wobei die zwei oder mehreren Kunststofffolien miteinander verschweißt oder verklebt sind, und wobei bevorzugt die Gießform und/oder die Leitung aus PETG-Folie und/oder Polyamid-Folie und/oder PE-Folie gefertigt ist oder sind.

Hierdurch kann die Gießform zweckmäßig und kostengünstig gefertigt werden. Die genannten Materialien sind zur Formgebung des Spacers aus dem Knochenzementteig geeignet. Zudem lassen sich die Spacer so nach dem Aushärten gut entformen.

Unter dem Begriff "Kunststofffolie" werden flächige Kunststoffe verstanden, die eine Schichtdicke bis zu 2 mm haben, bevorzugt eine Schichtdicke bis zu 1 mm haben. Die Kunststofffolien der Gießformen können durch Kalandrieren oder auch durch Spritzgießen hergestellt sein. Für die Formgebung der Gießformen kommen Tiefziehen und Kunststoffspritzgießen in Betracht. Die Gießformteile könne durch Ultraschallverschweißung, durch Hochfrequenzverschweißung, thermischer Verschweißung oder durch Klebung miteinander verbunden sein. Die Gießformteile können hierzu über Flansche miteinander verbunden sein. Es sind auch Kombinationen der Fügeverfahren möglich. Die Breite der Schweißnähte oder die Breite der Klebverbindung sollten zumindest 1 mm, bevorzugt zumindest 2 mm und besonders bevorzugt zumindest 3 mm sein. Dadurch werden druckfeste Gießformen aus kostengünstigen Kunststoffformen erhalten, die einem Druck von 10 N/cm² über einen Zeitraum von wenigen Minuten widerstehen, ohne dass die Fügenähte aufplatzen.

Alternativ können die Teile der Gießform auch mechanisch, zum Beispiel durch Klammern, miteinander verbunden werden. PETG ist ein mit Glykol modifiziertes Polyethylenterephthalat (PET), welches sich durch seine wässrigen Eigenschaften (Viskosität) auszeichnet und für den Spritzguss besonders geeignet ist.

Für die Herstellung der Gießformen können Kunststofffolien aus PETG, Polyamid und Polyethylen (PE) mit einer Schichtdicke von 500 µm bis 2000 µm verwendet werden. Dadurch ist es möglich, Gießformen herzustellen, die einen Druck von 10 N/cm² für eine Zeitraum von wenigen Minuten ohne Aufreißen tolerieren.

Bevorzugt kann auch vorgesehen sein, dass die Einfüllöffnung an einem Teil der Gießform angeordnet ist, der den Schaft des Spacers formt, vorzugsweise an einem Teil der Gießform angeordnet ist, der das distale Ende des Schafts des Spacers formt.

Hierdurch wird erreicht, dass der Anguss an einer Stelle des Spacers, nämlich dem Schaft angeordnet ist, an dem er im beim Patienten eingesetzten Zustand bei der Behandlung nicht oder weniger stört. Die Gleitflächen an den Köpfen können so glatt und ebenmäßig geformt sein, so dass weniger Abrieb und dadurch weniger Irritationen am umliegenden Gewebe entstehen.

Des Weiteren kann vorgesehen sein, dass die Gießform und/oder die Leitung zumindest im verformbaren Bereich elastisch und/oder plastisch verformbar ist oder sind, wobei bevorzugt die Gießform und/oder die Leitung zumindest im verformbaren Bereich plastisch verformbar ist.

Hiermit wird erreicht, dass die Verformung des verformbaren Bereichs zum Abtrennen des Knochenzementteigs in der Leitung ausreicht und möglich ist.

Ferner kann vorgesehen sein, dass der Anschluss zum flüssigkeitsdichten Verbinden einer Knochenzementkartusche ein Gewinde aufweist, insbesondere ein Innengewinde oder ein Außengewinde aufweist, wobei vorzugsweise ein Adapterelement der Knochenzementkartusche oder an der Knochenzementkartusche ein zu dem Gewinde passendes Gegengewinde aufweist.

Hiermit kann eine stabile und flüssigkeitsdichte Verbindung der Knochenzementkartusche zum Anschluss mit einfachen und kostengünstigen Mitteln aufgebaut werden. Zudem ist die Verbindung ohne weiteres lösbar. Durch die Verbindung über das Gewinde und das Gegengewinde kann Knochenzementteig mit hohem Druck, insbesondere hoch viskoser Knochenzementteig, aus der Knochenzementkartusche in die Gießform gepresst werden ohne das Knochenzementteig austritt und ohne dass die Gießform sich von der Knochenzementkartusche infolge des Drucks ablöst.

Es kann auch vorgesehen sein, dass die Klemmeinrichtung eine Platte mit einem sich zu einem Inneren der Platte hin verjüngenden Einschnitt ist, wobei der Einschnitt in einen Schlitz mündet, wobei die Breite des Schlitzes kleiner ist als die Dicke der Leitung in dem verformbaren Bereich und durch Einführen des verformbaren Bereichs in den Schlitz das Klemmen der Leitung in dem verformbaren Bereich erfolgt, wobei die Platte bevorzugt einteilig ist, oder die Klemmeinrichtung zweiteilig oder mehrteilig ist und zumindest zwei der Teile gegeneinander beweglich sind, um die Leitung in dem verformbaren Bereich zu Klemmen und/oder zu Knicken, wobei bevorzugt die Klemmeinrichtung eine Schraubklemme oder eine Verschlussklemme mit einer Rastvorrichtung ist.

Diese Klemmeinrichtungen sind kostengünstig zu realisieren und gleichzeitig einfach und unkompliziert in der Anwendung. Es können prinzipiell auch alle anderen Klemmeinrichtungen eingesetzt werden, mit denen die Leitung flüssigkeitsundurchlässig neben der Einfüllöffnung verschlossen werden kann.

Des Weiteren kann vorgesehen sein, dass der Anschluss aus thermoplastischem Kunststoff besteht, wobei vorzugsweise der Anschluss mit der Leitung und/oder der Gießform verklebt oder verschweißt ist.

Hierdurch kann die Vorrichtung kostengünstig gehalten werden und nach der Anwendung im OP-Bereich hygienisch durch Verbrennen entsorgt werden. Der Anschluss ist bevorzugt aus dem gleichen Kunststoff gefertigt wie die Gießform. Besonders geeignet ist der Kunststoff PETG zur Herstellung der Gießform und des Anschlusses.

Es kann bevorzugt vorgesehen sein, dass die Gießform ausgehend von dem Innenraum der Gießform mindestens drei oder vier Kavitäten zur Aufnahme von Haltestiften aufweist, wobei vorzugsweise die Gießform zweiteilig ist und die Kavitäten in Rändern oder in Flanschen zumindest eines Teils der zweiteiligen Gießform angeordnet sind.

Mit diesen Kavitäten kann ein Metallkern als Armierung in dem Spacer angeordnet und genau platziert werden. Die Haltestifte können vorzugsweise aus Polymethylmethacrylat gebildet sein. Diese können sich mit dem Zementteig verbinden. Nach der Aushärtung des eingebrachten Knochenzementteigs können diese einfach an der Spaceroberfläche abgeschnitten werden.

Ferner kann vorgesehen sein, dass die Vorrichtung einen Metallkern aufweist, der im Innenraum der Gießform anzuordnen ist, wobei bevorzugt der Metallkern Bohrungen zur Aufnahme von Haltestiften besitzt, wobei besonders bevorzugt die Bohrungen nicht in einem Gelenkkopf-Formteil der Gießform zum Formen einer Gleitfläche des Spacers angeordnet sind, ganz besonders bevorzugt die Bohrungen in einem Schaft-Formteil der Gießform zum Formen eines Schafts des Spacers angeordnet sind.

Der Schaft des Spacers dient der Verbindung zum Knochen und wird bei einer Implantation mit Knochenzement abgedeckt.

Vorzugsweise besteht der Metallkern aus einem biokompatiblen Metall oder aus einer biokompatiblen Metalllegierung, besonders bevorzugt aus chirurgischem Stahl. Besonders bevorzugt besteht der Metallkern aus Implantat-Stahl (316L).

Es kann auch vorgesehen sein, dass die Vorrichtung zumindest drei oder vier Haltestifte zur Halterung des Metallkerns in der Gießform aufweist.

Der Metallkern dient der Stabilisierung des Spacers und damit einer besseren Belastbarkeit des behandelten Gelenks.

Durch die Haltestifte wird der Metallkern in einer definierten Position innerhalb der Gießform gehalten. Dadurch wird die Dicke des Knochenzementmantels um den Metallkern definiert. Die Haltestifte sind vorzugsweise aus einem biokompatiblen Kunststoff gefertigt. Besonders eignet sich hierbei Polymethylmethacrylat. Haltestifte aus Polymethylmethacrylat verbinden sich mit dem Knochenzementteig irreversibel. Nach Aushärtung des Knochenzementteigs werden die aus dem Spacer herausragenden Haltestifte einfach abgeschnitten. Die im Inneren des Spacer befindlichen Reste der Haltestifte verbleiben dort.

Des Weiteren kann vorgesehen sein, dass in der Gießform zumindest eine Entlüftungsöffnung vorgesehen ist, durch die Luft oder Gas aus dem Innenraum der Gießform entweichen kann, wobei bevorzugt wenigstens eine der zumindest einen Entlüftungsöffnung durch einen Spalt geformt ist, der zwischen zwei Teilen der Gießform, insbesondere zwischen zwei Flanschen der Gießform, beim Anlegen der Teile aneinander verbleibt.

Hiermit kann Luft oder Gas aus dem Inneren der Gießform entweichen, wenn der Knochenzementteig eingefüllt wird. Dadurch können Lufteinschlüsse im Spacer und dadurch Unebenheiten an der Oberfläche des Spacers vermieden werden.

Die der vorliegenden Erfindung zugrundeliegenden Aufgaben werden auch gelöst durch ein Verfahren zur Herstellung eines Spacers, der zum temporären Ersetzen eines Gelenks oder eines Teils eines Gelenks, insbesondere eines Hüftgelenks oder eines Schultergelenks, geeignet ist, der Spacer aufweisend eine artikulierende Oberfläche des Gelenks, das Verfahren aufweisend die folgenden chronologischen Schritte:
A) Bereitstellen einer Gießform mit einem Anschluss zum flüssigkeitsdichten Anschließen einer Knochenzementkartusche und einer Leitung, die den Anschluss für den Knochenzementteig durchlässig mit einem Innenraum der Gießform verbindet;
B) Einpressen von Knochenzementteig durch den Anschluss und durch die Leitung hindurch in den Innenraum der Gießform, bis der Innenraum der Gießform mit Knochenzementteig gefüllt ist;
C) Knicken oder Klemmen der Leitung, während fluider Knochenzementteig in der Leitung enthalten ist, wobei sich der freie Leitungsquerschnitt oder die Querschnittsfläche der Leitung durch das Knicken oder Klemmen um mindestens 90% reduziert;
D) Aushärten des Knochenzementteigs in der Gießform; und
E) Entnehmen des so geformten und ausgehärteten Spacers aus der Gießform.

Der Spacer ist für medizinische Anwendungen vorgesehen. Das erfindungsgemäße Verfahren umfasst nicht die Implantation bei einem Patienten sondern lediglich das Ausformen des Spacers.

Durch das Knicken oder Klemmen der Leitung wird der Knochenzementteig in zwei Teile geteilt oder es wird eine Sollbruchstelle mit verringerter Querschnittsfläche oder verringerte Dicke erzeugt, die sich nach dem Aushärten leicht abbrechen oder abschneiden lässt und nicht gesägt werden muss.

Das Klemmen der Leitung erfolgt erfindungsgemäß bevorzugt mit einer Klemmeinrichtung.

Nach Schritt E) kann der Spacer entgratet, geglättet, geschliffen, gereinigt, poliert und/oder bereichsweise aufgeraut werden.

Zum Entnehmen des geformten und ausgehärteten Spacers aus der Gießform in Schritt E) kann die Gießform nach Schritt D) geöffnet werden.

Bei erfindungsgemäßen Verfahren kann vorgesehen sein, dass das Verfahren mit einer oben genannten erfindungsgemäßen Vorrichtung durchgeführt wird.

Hierdurch kann das Verfahren die zuvor beschriebenen Vorteile der erfindungsgemäßen Vorrichtung nutzen.

Des Weiteren kann vorgesehen sein, dass vor Schritt B) eine Knochenzementkartusche an den Anschluss angeschlossen wird, insbesondere in den Anschluss eingeschraubt wird, und in Schritt B) das Einpressen des Knochenzementteigs aus der Knochenzementkartusche erfolgt, wobei bevorzugt zuvor der Knochenzementteig durch Mischen von Ausgangskomponenten, insbesondere einer Monomerflüssigkeit und einer Knochenzementpulvers, innerhalb der Knochenzementkartusche hergestellt wird und besonders bevorzugt die Knochenzementkartusche nach Schritt C) von dem Anschluss getrennt wird, insbesondere aus dem Anschluss herausgeschraubt wird.

Hiermit wird das Verfahren weiter komplettiert und in der Anwendung vereinfacht.

Des Weiteren kann vorgesehen sein, dass vor Schritt B) der Knochenzementteig in der Knochenzementkartusche aus einer Monomerflüssigkeit und aus einem Zementpulver gemischt wird.

Hierdurch kann zur Herstellung des Spacers ein frisch gemischter Knochenzementteig verwendet werden. Insbesondere PMMA-Knochenzementteige lassen sich im gemischten Zustand nur schwer oder gar nicht über Zeiträume von mehr als wenigen Minuten lagern. Zudem können dem Knochenzementteig so auch kurz vor der Herstellung des Spacers dem Knochenzementteig zur Behandlung geeignete pharmazeutische Wirkstoffe, wie Antibiotika und Antimykotika, beigemengt werden.

Ferner kann vorgesehen sein, dass zum flüssigkeitsdichten Verbinden der Knochenzementkartusche mit dem Anschluss die Knochenzementkartusche hineingedreht oder eingeschraubt wird und bevorzugt zum Lösen der Knochenzementkartusche von dem Anschluss die Knochenzementkartusche oder die neue Knochenzementkartusche aus dem Anschluss herausgedreht oder herausgeschraubt wird.

Neben dem Schrauben kann die Knochenzementkartusche beispielsweise mit einem Bajonettverschluss mit dem Anschluss verbunden werden.

Durch das Eindrehen oder Einschrauben der Knochenzementkartusche in den Anschluss kann eine flüssigkeitsdichte Verbindung zwischen dem Anschluss und der Knochenzementkartusche bereitgestellt werden.

Ferner kann vorgesehen sein, dass das Einpressen des Knochenzementteigs mit Hilfe einer Auspressvorrichtung erfolgt, wobei bevorzugt die Auspressvorrichtung einen Kolben in der Knochenzementkartusche antreibt.

Hierdurch kann eine übliche Knochenzementkartusche zum Herstellen des Knochenzementteigs und zum Einpressen des Knochenzementteigs in die Gießform verwendet werden.

Auch kann vorgesehen sein, dass in Schritt B) die in der Gießform enthaltene Luft durch zumindest eine Entlüftungsöffnung in die Gießform verdrängt wird, während der Knochenzementteig in die Gießform eingepresst wird, wobei vorzugsweise die zumindest eine Entlüftungsöffnung für den Knochenzementteig undurchlässig ist.

Hiermit wird verhindert, dass Lufteinschlüsse in der Gießform verbleiben, die die Oberfläche des Spacers oder die Stabilität des Spacers beeinträchtigen könnten.

Des Weiteren kann vorgesehen sein, dass vor Schritt B), und vorzugsweise vor Schritt A), ein Metallkern innerhalb der Gießform angeordnet wird, wobei bevorzugt der Metallkern über mehrere Haltestifte von einer Innenwand der Gießform beabstandet wird, wobei besonders bevorzugt die mehreren Haltestifte in Bohrungen im Metallkern und in Kavitäten zur Aufnahme der Haltestifte in der Innenwand der Gießform befestigt werden.

Hierdurch kann der Spacer mit Hilfe der Vorrichtung mit einer innenliegenden Armierung aufgebaut werden. Der Knochenzementteig umfließt dabei den in der Gießform angeordneten Metallkern. Hiermit wird die mechanische Stabilität des Spacers erhöht.

Bevorzugt kann auch vorgesehen sein, dass durch das Knicken oder Klemmen in Schritt C) die Leitung für den Knochenzementteig undurchlässig verschlossen wird, vorzugsweise flüssigkeitsdicht verschlossen wird.

Dadurch wird sichergestellt, dass der Knochenzementteig beim Aushärten in der Gießform verbleibt.

Schließlich kann vorgesehen sein, dass die Gießform zumindest zwei Teile aufweist und dass nach Schritt D) und vor Schritt E) ein Schritt D2) erfolgt: D2) Öffnen der Gießform durch Trennen der zumindest zwei Teile der Gießform voneinander.

Hierdurch wird die Anwendung der Gießform vereinfacht.

Der Erfindung liegt die überraschende Erkenntnis zugrunde, dass es durch den verformbaren Bereich der Leitung sowie mit der Sollknickstelle und/oder der Klemmeinrichtung gelingt, eine Vorrichtung zum Herstellen von Spacern bereitzustellen, bei der mit einfachsten Mitteln ein Abtrennen eines Angusses und damit ein Abtrennen eines Knochenzementteigs nach dem Befüllen einer Gießform erfolgen kann. Das Abtrennen kann ohne aufwendige technische Hilfsmittel erfolgen und auch von Hilfspersonal ohne Vorkenntnisse umgesetzt werden. Zudem werden keine aufwendige Laborausrüstungen oder irgendwelche anderen Hilfsmittel benötigt. Die Vorrichtung ist theoretisch sogar von nicht ausgebildetem Hilfspersonal fehlerfrei einsetzbar. Wesentlich ist dabei, dass der verformbare Bereich der Leitung derart stark verformt werden kann, dass er für den verwendeten Knochenzementteig undurchlässig wird oder bevorzugt die Leitung in dem verformbaren Bereich sogar vollständig geschlossen werden kann, und zwar, ohne dass die Wandungen der Leitung aufbrechen oder aufreißen. Der verformbare Bereich soll also zerstörungsfrei verformbar sein.

Mit Hilfe der Klemmeinrichtung oder durch Knicken kann nach erfolgter Füllung der Gießform mit Knochenzementteig, bevor die Zementkartusche von der Gießform getrennt wird, der verformbare Bereich der Leitung (insbesondere vorformbare Bereich der Gießform, der die Leitung bildet) unmittelbar neben der Einfüllöffnung zusammengepresst werden oder auch geknickt werden, so dass die im Leitungsinnenwände oder die Gießforminnenwände bereichsweise aufeinander liegen und damit die Leitung beziehungsweise die Gießform flüssigkeitsundurchlässig verschließen. Der Knochenzementteig ist im Innenraum der Gießform dadurch eingeschlossen und kann nach Entfernen der Knochenzementkartusche, die zum Befüllen des Innenraums der Gießform verwendet wird, nicht aus der Gießform ausfließen.

Vorteilhaft ist dabei, dass die Gießform verschlossen werden kann, ohne dass Öffnungen in der Gießformwand zum Verschluss notwendig sind. Dadurch werden Abdichtungsprobleme der Gießform beim Einpressen von Knochenzementteig mit hohem Druck vermieden. Beim Einpressen von Knochenzementteig mit hohem Druck kann dieser schon bei geringen Undichtigkeiten aus der Gießform austreten.

Dieser verformbare Bereich der Gießform kann mit Klemmen oder Knickeinrichtungen unterschiedlicher Bauart zusammengepresst werden.

Durch das Zusammenpressen der Gießform neben der Einfüllöffnung wird der Knochenzementteig in diesem Bereich ebenfalls zusammengedrückt. Nach erfolgter Aushärtung kann der Hüft- oder Schulterspacer problemlos über diesen eingeschnürten Bereich vom Anguss abgebrochen werden, sofern überhaupt noch eine Verbindung zu dem Knochenzementteig in der restlichen Leitung verbleibt.

Knochenzementteig, insbesondere nicht hochviskoser Knochenzementteig, kann durch den Verschluss der Leitung nicht aus der Gießform fließen. Eine Ausbildung von Fehlstellen im Spacer infolge des Auslaufens von Knochenzementteig wird dadurch verhindert. Weiterhin wird durch die erfindungsgemäßen Maßnahmen der noch in der Knochenzementkartusche befindliche Rest des Knochenzementteigs vom Knochenzementteig in der Gießform getrennt. Nach beendeter Aushärtung des Knochenzementteigs ist es daher nicht mehr notwendig, den Anguss aufwendig mechanisch beispielsweise durch Sägen abzutrennen. Eventuell verbliebene dünne Verbindungen können leicht abgebrochen oder abgeschnitten werden. Dadurch wird der Arbeits- und Zeitaufwand für das OP-Personal gesenkt.

Der Anguss des Spacers wird durch die Einfüllöffnung in der angrenzenden Leitung gebildet. Durch das Klemmen oder Knicken der Leitung in dem verformbaren Bereich wird die Gießform für Knochenzementteig undurchlässig verschlossen. Das bedeutet, der verformbare Bereich der Leitung hat gleichzeitig eine Ventilfunktion. Komplexe zusätzliche Ventile sind nicht notwendig.

Ein mit der Vorrichtung hergestellter Spacer kann vorteilhaft im Rahmen von zweizeitigen septischen Revisionen verwendet werden, bei denen eine Infektion mit zwei oder mehreren mikrobiellen Keimen und insbesondere mit problematischen Keimen vorliegt.

Eine beispielhafte erfindungsgemäße Vorrichtung kann zusammengesetzt sein aus
a) einer Knochenzementkartusche zum Mischen von Polymethylmethacrylat-Knochenzement-Komponenten und zum Austragen von gemischtem Polymethylmethacrylat-Knochenzementteig,
b) einem Adapterelement, das mit dem Kartuschenkopf der Knochenzementkartusche verbunden ist oder verbindbar ist, wobei das Adapterelement eine reversible formschlüssige Verbindung ausbilden kann und wobei das Adapterelement eine Durchführung besitzt, die den Innenraum der Zementkartusche flüssigkeitsdurchlässig mit der Umgebung verbindet,
c) einer aus Kunststofffolie gefertigten hohlen und plastisch verformbaren Gießform, die eine Einfüllöffnung zum Eintrag des Knochenzementteigs besitzt,
d) einem formstabilen Anschluss,
d1) der in der Leitung zur Einfüllöffnung der Gießform zum Eintrag des Knochenzementteigs angeordnet ist,
d2) wobei der formstabile Anschluss flüssigkeitsundurchlässig mit der Gießformwand verbunden ist,
d3) wobei der formstabile Anschluss eine Durchführung besitzt, die den Innenraum der Gießform mit der Umgebung flüssigkeitsdurchlässig verbindet, und
d4) wobei das Adapterelement reversibel formschlüssig mit dem Anschluss verbunden ist oder verbindbar ist,
e) einen von außen zusammenpressbaren oder knickbaren Abschnitt der Gießform, der eine Leitung bildet und der der Einfüllöffnung der Gießform unmittelbar benachbart ist, und
f1) einer einteiligen oder mehrteiligen Klemmeinrichtung, die den zusammenpressbaren Abschnitt der Gießform von außen durch Zusammenpressen flüssigkeitsdicht verschließt oder verschließen kann, oder
f2) eine Sollknickstelle, die bei Knicken den zusammenpressbaren Abschnitt der Gießform von außen durch Zusammenpressen flüssigkeitsdicht verschließt oder verschließen kann.

Die Einfüllöffnung ist bevorzugt in dem Bereich des (distalen) Schaftendes der Hüft- und Schulterspacergießform angeordnet.

Mit Hilfe der Klemmeinrichtung oder durch Knicken nach erfolgter Füllung der Gießform mit Knochenzementteig wird, bevor die Knochenzementkartusche von der Gießform getrennt wird, der zusammenpressbare Bereich der Gießform unmittelbar neben der Einfüllöffnung zusammengepresst oder geknickt, so dass die im Gießforminnenwände aufeinander liegen und damit die Gießform flüssigkeitsundurchlässig verschließen. Der Knochenzementteig ist im Hohlraum der Gießform dadurch eingeschlossen und kann nach Abtrennung der Zementkartusche nicht aus der Gießform ausfließen.

Ein beispielhaftes erfindungsgemäßes Verfahren zur Herstellung von Spacern mit der erfindungsgemäßen Vorrichtung kann die folgenden nacheinander ablaufenden Schritte umfassen:
a) Bereitstellen der Gießform, die eine Einfüllöffnung, einen Anschluss zum Einspeisen von Knochenzementteig und einen verformbaren Bereich der Leitung aufweist,
b) Mischen des Knochenzementpulvers mit der Monomerflüssigkeit in einer Knochenzementkartusche bis sich ein Knochenzementteig gebildet hat, wobei die Knochenzementkartusche ein Adapterelement am Kartuschenkopf besitzt, das flüssigkeitsdurchlässig mit dem Innenraum der Kartusche verbunden ist,
c) Flüssigkeitsdurchlässiges Verbinden der Knochenzementkartusche mit der Gießform durch Einschrauben des Adapterelements in ein Innengewinde des Anschlusses,
d) Einpressen des Knochenzementteigs aus der Knochenzementkartusche in die Gießform mit Hilfe einer Auspressvorrichtung, wobei der Knochenzementteig die Luft im Hohlraum der Gießform verdrängt und vorzugsweise die verdrängte Luft durch mindestens eine Entlüftungsöffnung aus dem Hohlraum der Gießform in die Umgebung austritt,
e) Flüssigkeitsdichtes Verschließen der Einfüllöffnung durch Zusammenpressen des verformbaren Bereichs der Leitung der Gießform neben der Einfüllöffnung,
f) Herausschrauben der Knochenzementkartusche mit dem Adapterelement aus dem Anschluss,
g) Trennen der Knochenzementkartusche von der Gießform
h) Aushärten des Knochenzementteigs in der Gießform,
i) Öffnen der Gießform nach erfolgter Aushärtung des Knochenzementteigs und
j) Entnahme des Spacers.

Der Anschluss kann eine Schraubenmutter in der Gießformwand sein. In der Gießform kann ein Metallkern mit Haltestiften in der Gießform gehalten werden.

Erfindungsgemäß ist weiterhin ein Verfahren zum Verschließen der Gießform der Vorrichtung (des Kits), wobei bei diesem Verfahren die Gießform beziehungsweise die Leitung im verformbaren Bereich durch Zusammenpressen der verformbaren Gießformwände oder der verformbaren Leitungswände durch Knicken oder mit einer Klemmvorrichtung, die an der Außenseite der Gießformwände angreift, flüssigkeitsundurchlässig verschlossen wird.

Im Folgenden werden weitere Ausführungsbeispiele der Erfindung anhand von sechzehn schematisch dargestellten Figuren erläutert, ohne jedoch dabei die Erfindung zu beschränken. Dabei zeigt:
Figur 1: eine schematische perspektivische Querschnittansicht einer beispielhaften erfindungsgemäßen Vorrichtung zum Herstellen eines Hüftgelenkspacers;
Figur 2: eine schematische perspektivische Außenansicht der Vorrichtung nach Figur 1;
Figur 3: eine schematische perspektivische Ansicht einer offenen Hälfte der Gießform nach den Figuren 1 und 2;
Figur 4: eine schematische perspektivische Ansicht der offenen Hälfte der Gießform nach Figur 3 mit eingelegtem Metallkern;
Figur 5: eine schematische Querschnittansicht der Vorrichtung nach den Figuren 1 bis 4 vor dem Anschließen einer Knochenzementkartusche;
Figur 6: eine schematische perspektivische Querschnittansicht der Vorrichtung mit angeschlossener Knochenzementkartusche;
Figur 7: eine schematische Querschnittansicht der Vorrichtung während dem Einfüllen von Knochenzementteig;
Figur 8: eine schematische Querschnittansicht der Vorrichtung mit eingefülltem Knochenzementteig;
Figur 9: eine schematische perspektivische Querschnittansicht der Vorrichtung vor dem Klemmen eines verformbaren Bereichs der Leitung mit einer ersten Klemmeinrichtung;
Figur 10: eine schematische Querschnittansicht der Vorrichtung nach dem Klemmen des verformbaren Bereichs der Leitung;
Figur 11: eine schematische perspektivische Querschnittansicht der Vorrichtung nach dem Klemmen des verformbaren Bereichs der Leitung;
Figur 12: eine schematische perspektivische Ansicht auf einen Metallkern für eine erfindungsgemäße Vorrichtung zur Herstellung eines Hüftgelenkspacers;
Figur 13: eine schematische perspektivische Querschnittansicht einer beispielhaften zweiten erfindungsgemäßen Vorrichtung zum Herstellen eines Hüftgelenkspacers vor dem Klemmen eines verformbaren Bereichs der Leitung mit einer alternativen zweiten Klemmeinrichtung;
Figur 14: eine schematische perspektivische Querschnittansicht der Vorrichtung nach Figur 13 nach dem Klemmen des verformbaren Bereichs der Leitung;
Figur 15: eine schematische Querschnittansicht der Vorrichtung nach den Figuren 13 und 14 nach dem Klemmen des verformbaren Bereichs der Leitung; und
Figur 16: eine perspektivische Ansicht auf einen Spacer, der mit einer erfindungsgemäßen Vorrichtung hergestellt wurde.

In den Figuren werden auch für unterschiedliche Ausführungsbeispiele die gleichen Bezugszeichen verwendet, da sich die Ausführungsbeispiele nur geringfügig unterscheiden und gleichzeitig die Vergleichbarkeit der Ausführungsbeispiele verbessert wird.

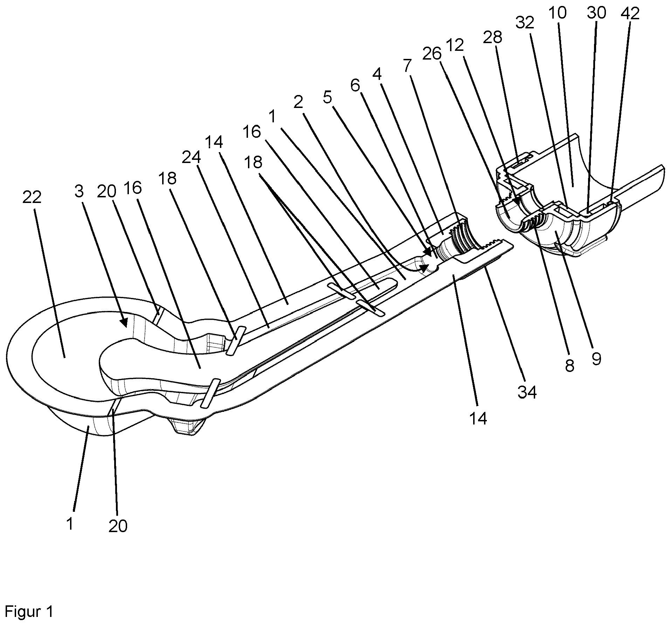

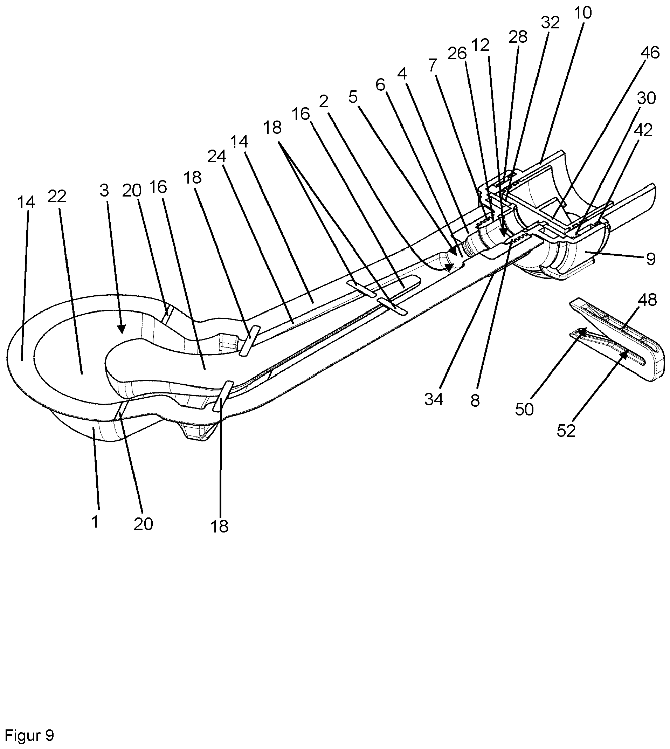

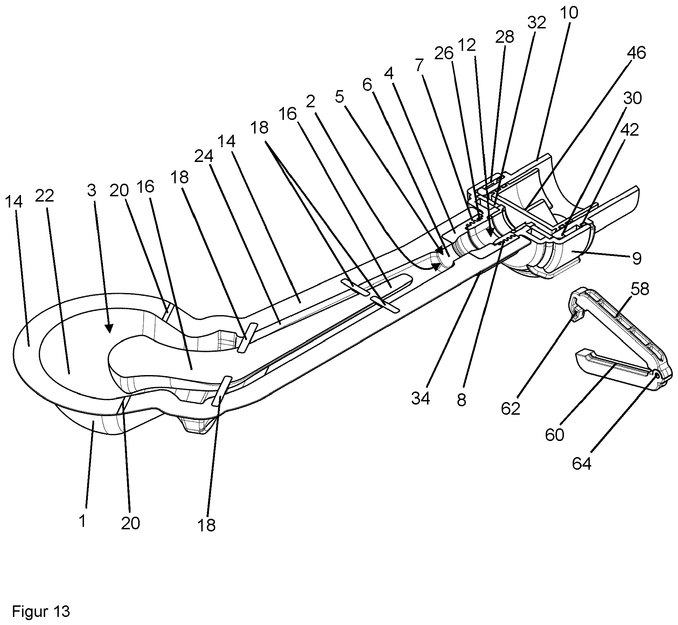

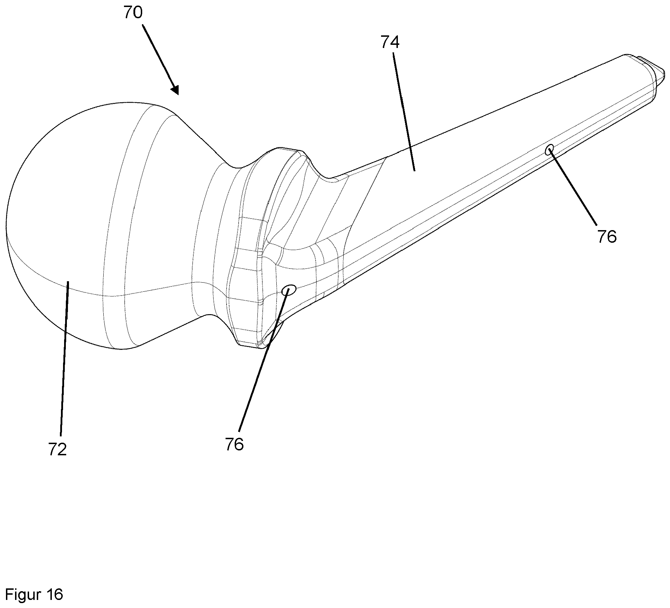

In den Figuren 1 bis 12 sind Abbildungen eines ersten Ausführungsbeispiels einer erfindungsgemäßen Vorrichtung zur Herstellung eines Hüftgelenkspacers und Teile davon in verschiedenen Ansichten gezeigt. Die Figuren 13 bis 15 zeigen ein im wesentlichen gleiches Ausführungsbeispiel jedoch mit einer anderen Klemmeinrichtung 58. Figur 16 zeigt einen mit einer erfindungsgemäßen Vorrichtung hergestellten Spacer 70 beziehungsweise Hüftgelenkspacer.

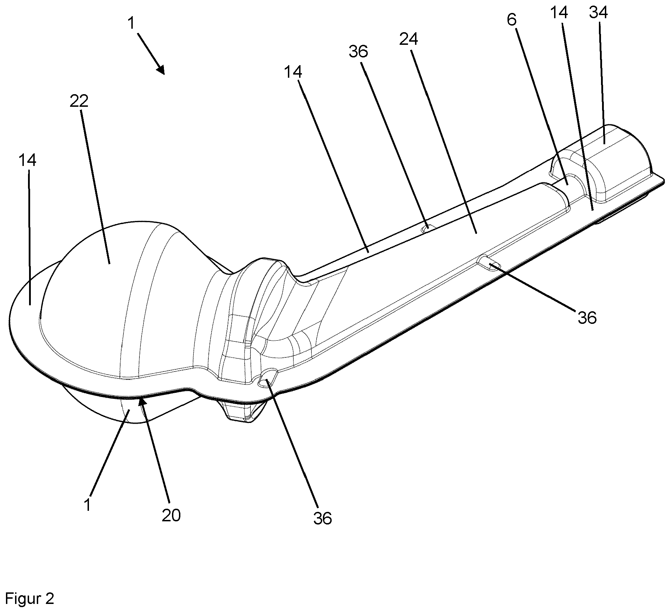

Die erste erfindungsgemäße Vorrichtung ist zur Herstellung eines Spacers für ein Hüftgelenk geeignet und vorgesehen. Die Vorrichtung umfasst eine Gießform 1. Die Gießform 1 kann zweiteilig aufgebaut sein. In den Figuren 1 und 3 bis 11 und 13 bis 15 ist jeweils nur einer der beiden Teile der Gießform 1 erkennbar. In Figur 2 sind beiden Teile der Gießform 1 dargestellt. An einer Seite der Gießform 1 kann eine Einfüllöffnung 2 zum Einfüllen von Knochenzementteig 40 in einen von der Gießform 1 begrenzten Innenraum 3 der Gießform 1 ausgeformt sein. Die Gießform 1 kann aus einer Kunststofffolie aus PETG geformt sein oder aus einem Verbund mehrerer Kunststofffolien, die miteinander laminiert sind. Die Gießform 1 kann plastisch verformbar sein. Die Vorrichtung kann einen formstabilen Anschluss 4 zum Anschließen einer Knochenzementkartusche 10 aufweisen. Der Anschluss 4 kann aus einem thermoplastischen Kunststoff bestehen und kann mit der Gießform 1 flüssigkeitsdicht verschweißt oder verklebt sein, so dass kein Knochenzementteig 40 zwischen dem Anschluss 4 und der Gießform 1 herausgedrückt werden kann, wenn der Knochenzementteig 40 unter Druck mit der Knochenzementkartusche 10 in den Innenraum 3 der Gießform 1 gepresst wird.

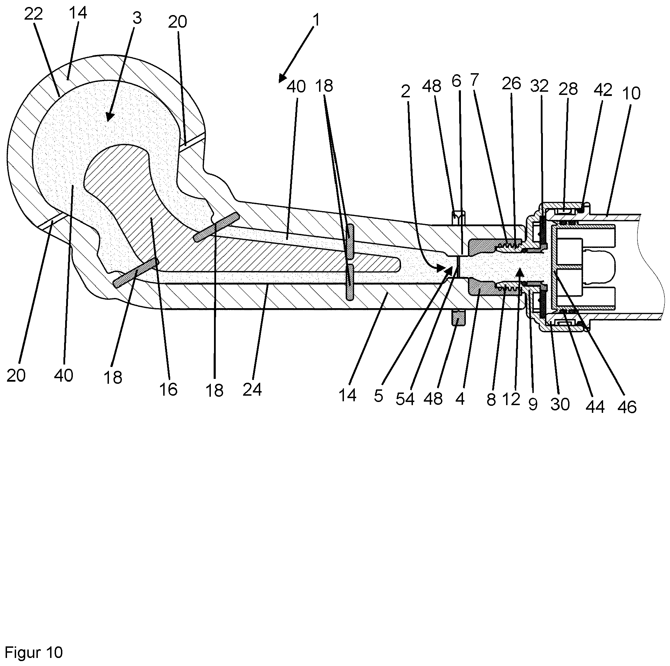

Ausgehend von der Einfüllöffnung 2 kann eine Leitung 5 durch die beiden Teile der Gießform 1 geformt beziehungsweise begrenzt sein, die durch eine in beiden Teilen der Gießform 1 jeweils Halbkreisförmige zylindrische Öffnung definiert ist. Die Leitung 5 verbindet die Einfüllöffnung 2 mit dem Anschluss 4. Der Anschluss 4 weist einen für den Knochenzementteig 40 durchlässigen Durchgang auf, der in die Leitung 5 mündet. Die Wandungen der Gießform 1 können in dem gesamten Bereich, in dem die Leitung 5 ausgeformt ist, verformbar sein. Dadurch wird ein verformbarer Bereich 6 der Leitung 5 gebildet. Der verformbare Bereich 6 der Leitung 5 kann sich über die gesamte Leitung 5 erstrecken. Der verformbare Bereich 6 der Leitung 5 kann elastisch oder plastisch derart verformbar sein, dass die Wandungen der Leitung 5 bei einem Knicken senkrecht zur Achse der Leitung 5 um mindestens 100° oder bei einem Klemmen des verformbaren Bereichs 6 (siehe Figuren 10, 11, 14 und 15) nicht reißen oder brechen, auch wenn die Wandungen aufeinander gepresst werden und so die Leitung 5 in dem verformbaren Bereich 6 verschließen. Der verformbare Bereich 6 der Leitung 5 bildet eine mechanische Schwachstelle zwischen dem Anschluss und dem den Innenraum 3 formgebenden Teil der Gießform 1. Bei einem Knicken beziehungsweise Falten des Anschlusses 4 gegen diesen, den Innenraum 3 formgebenden Teil der Gießform 1 wird die Vorrichtung also im verformbaren Bereich 6 knicken und sich dort plastisch verformen. Sofern ausreichend weit geknickt wird, werden die Innenwände der Leitung 5 in dem verformbaren Bereich dadurch aneinander gedrückt und die Leitung 5 für den Knochenzementteig 40 undurchlässig verschlossen.

In dem Anschluss 4 kann ein Gewinde 7 in Form eines Innengewindes angeordnet sein. Das Gewinde 7 des Anschlusses 4 kann mit einem Gegengewinde 8 in Form eines passenden Außengewindes an einem Adapterelement 9 der Knochenzementkartusche 10 lösbar verbunden werden. Das Gegengewinde 8 kann an einem zylindrischen Stutzen 26 des Adapterelements 9 angeordnet sein. Dadurch kann die Knochenzementkartusche 10 lösbar und flüssigkeitsdicht mit dem Anschluss 4 verbunden werden, indem die Knochenzementkartusche 10 mit dem Stutzen 26 in das Gewinde 7 des Anschlusses 4 eingeschraubt wird.

Die Knochenzementkartusche 10 kann an ihrer Vorderseite eine Austragsöffnung 12 zum Austragen des Knochenzementteigs 40 aus der Knochenzementkartusche 10 aufweisen. Die Austragsöffnung 12 kann in dem Stutzen 26 des Adapterelements 9 angeordnet sein und durch diesen begrenzt werden. Das Adapterelement 9 kann die Knochenzementkartusche 10 an ihrer Vorderseite bis auf die Austragsöffnung 12 und gegebenenfalls bis auf einen Vakuumanschluss (nicht zu sehen) verschließen.

Die Gießform 1 kann kostengünstig aus Kunststofffolie gefertigt werden. Die Kunststofffolie kann mehrere Schichten aufweisen. Die beiden Teile der Gießform 1 können über Flansche 14 aneinander befestigt werden. Durch Verbinden der beiden Teile der Gießform 1 über die Flansche 14 kann die Gießform 1 nach außen verschlossen werden.

Im Inneren der Gießform 1 kann ein Metallkern 16 platziert werden. Der Metallkern 16 kann aus chirurgischem Stahl oder aus Titan bestehen. Alternativ wäre es theoretisch auch möglich, den Metallkern 16 aus einem Kunststoff wie PMMA zu fertigen. Der Metallkern 16 kann über Haltestifte 18 mit der Gießform 1 verbunden werden. Der Metallkern 16 kann mit Hilfe der Haltestifte 18 von der Innenwand der Gießform 1 beabstandet sein, so dass der Knochenzementteig 40 den Metallkern 16 vollständig umfließen kann. Der Metallkern 16 bewirkt eine Stabilisierung des Spacers. Die Haltestifte 18 können aus PMMA bestehen. Dieses kann sich mit einem Knochenzementteig 40 aus PMMA irreversibel verbinden.

In der Gießform 1 können Entlüftungsöffnungen 20 angeordnet sein. Durch die Entlüftungsöffnungen 20 kann Luft oder Gas aus dem Inneren der geschlossenen Gießform 1 entweichen, wenn ein Knochenzementteig 40 durch die Einfüllöffnung 2 in den Innenraum 3 der Gießform 1 eingefüllt wird. Die Entlüftungsöffnungen 20 können durch eine schmale Vertiefung in den Flanschen 14 oder in einem der Flansche 14 realisiert werden. Die Entlüftungsöffnungen 20 können so schmal ausgeführt sein, dass der viskose Knochenzementteig 40 nicht durch die Entlüftungsöffnungen 20 fließen kann.

Die Gießform 1 kann einen Gelenkkopf-Formteil 22 zum Ausformen eines Kopfs 72 eines Spacers 70 und einen Schaft-Formteil 24 zum Ausformen eines Schafts 74 eines Spacers 70 (siehe analog Figur 16) aufweisen.

Das Adapterelement 9 kann über ein Rastmittel 28 am Adapterelement 9 mit einer Gegenrastung 30 an einer zylindrischen Wandung der Knochenzementkartusche 10 verbunden werden oder sein. Zur Abdichtung kann eine umlaufende Dichtung 42 vorgesehen sein, die die zylindrische Wandung der Knochenzementkartusche 10 gegen das Adapterelement 9 abdichtet.

In dem Adapterelement 9 kann ein Vakuumanschluss (nicht zu sehen) angeordnet sein, mit dem ein Innenraum der Knochenzementkartusche 10, in dem der Knochenzementteig 40 gemischt wird, evakuiert werden kann. Dadurch kann der Knochenzementteig 40 unter Vakuum gemischt werden. Zur Abdichtung kann in zwischen dem Vakuumanschluss und dem Innenraum der Knochenzementkartusche 10 eine Porenscheibe 32 angeordnet sein, die für Zementpulver und Knochenzementteig undurchlässig ist, aber für Luft durchlässig ist.

Der Anschluss 4 kann an einem distalen Ende des den Schaft 74 formenden Teils der Gießform 1 angeordnet sein. Um den Anschluss 4 mit der Gießform 1 zu verbinden, kann eine passende Form 34 für die Anschlussbefestigung vorgesehen sein. Die Form 34 zur Anschlussbefestigung kann formschlüssig und bündig mit dem Anschluss 4 verbunden sein. Im Bereich der Flansche 14 können in der Gießform 1 Formen 36 für Kavitäten 38 angeordnet sein, in denen die Haltestifte 18 angeordnet werden können, um den Metallkern 16 im Innenraum 3 der Gießform 1 zu halten. Die Haltestifte 18 können den Metallkern 16 derart im Innenraum 3 der Gießform 1 halten, dass er von den Innenwänden der Gießform 1 derart beabstandet ist, dass der Knochenzementteig 40 den Metallkern 16 im Innenraum 3 der Gießform 1 vollständig umfließen kann.

Im zylindrischen Innenraum der Knochenzementkartusche 10 kann ein Kolben 46 zum Austreiben des Knochenzementteigs 40 aus der Knochenzementkartusche 10 durch das Adapterelement 9, durch die Leitung 5 und durch die Einfüllöffnung 2 in den Innenraum 3 der Gießform 1 angeordnet sein. Hierzu kann der Kolben 46 an seiner Außenseite zylindrisch geformt und über zwei umlaufende Dichtungen 44 gegen den zylindrischen Innenraum abgedichtet sein. Durch Vortreiben des Kolbens 46 kann der Knochenzementteig 40 aus der Austragsöffnung 12 der Knochenzementkartusche 10 gepresst werden.

Die Porenscheibe 32 kann in dem Adapterelement 9 angeordnet sein. Die Porenscheibe 32 ist für den Knochenzementteig 40 und Knochenzementpulver und vorzugsweise auch für eine Monomerflüssigkeit als Ausgangskomponente des Knochenzementteigs 40 undurchlässig. Der Vakuumanschluss (nicht gezeigt) kann von der Porenscheibe 32 abgedeckt sein. Hierdurch wird verhindert, dass ein Knochenzementpulver als Ausgangskomponente des Knochenzementteigs 40 in den Vakuumanschluss eindringen kann.

Der verformbare Bereich 6 der Leitung 5 kann beispielsweise durch Knicken der Leitung 5 senkrecht zur Leitungsrichtung derart verformt werden, dass der innere Durchmesser der Leitung 5 und dabei die Querschnittsfläche der Leitung 5 zumindest so weit reduziert wird, dass der Knochenzementteig 40 nicht mehr durch die Leitung 5 fließen kann. Vorzugsweise werden die Innenwände der Leitung 5 beim Knicken im Knick aneinandergedrückt und dadurch die Leitung 5 verschlossen.

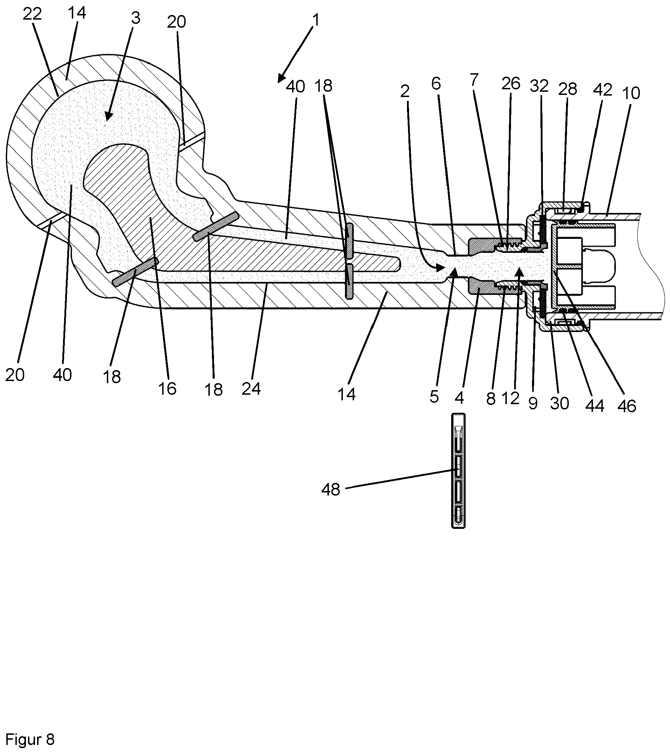

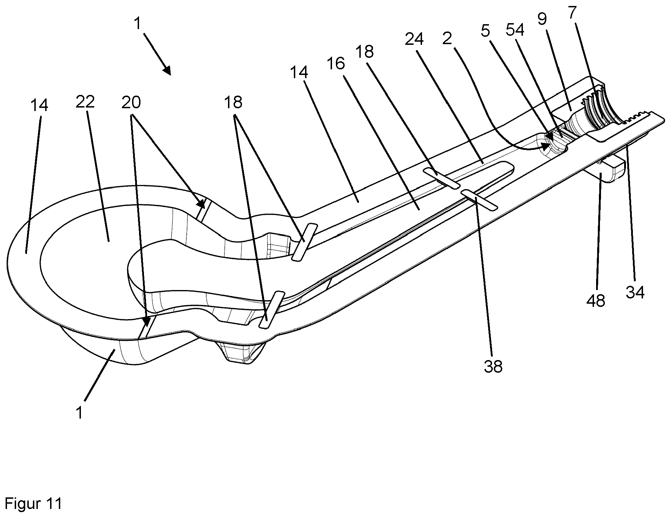

Alternativ kann der verformbare Bereich 6 der Leitung 5 auch mit Hilfe einer Klemmeinrichtung 48 geklemmt werden, wie sie in den Figuren 8 bis 11 gezeigt ist. Die Klemmeinrichtung 48 kann wie eine Platte aufgebaut sein, die einen sich zum inneren der Platte hin verjüngenden Einschnitt 50 aufweist. Der Einschnitt 50 kann in einen Schlitz 52 münden. Die Klemmeinrichtung 48 kann auf die Weise einteilig aufgebaut sein und als kostengünstiges einteiliges Kunststoffformteil aus thermoplastischem Kunststoff gefertigt werden. Um ein Klemmen der Leitung 5 zu ermöglichen, kann die Breite des Schlitzes 52 kleiner sein als die Dicke der Leitung 5 in dem verformbaren Bereich 6. Die Klemmeinrichtung 48 kann auf den verformbaren Bereich 6 der Leitung 5 aufgeschoben werden. Dabei wird der verformbare Bereich 6 in den Schlitz 52 eingedrückt und dabei die Leitung 5 in dem verformbaren Bereich 6 zusammengequetscht. Dadurch erfolgt ein Klemmen der Leitung 5 in dem verformbaren Bereich 6 und es entsteht ein verformter Bereich 54 der Leitung 5, der in den Figuren 10 und 11 gezeigt ist. Der verformte Bereich 54 bildet einen geklemmten Bereich der Leitung 5, durch die der Knochenzementteig 40 nicht hindurchfließen kann.

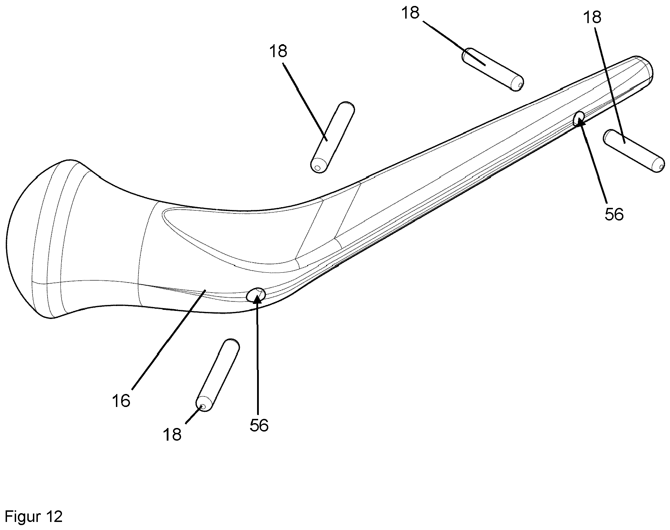

Figur 12 zeigt den Metallkern 16, der in der Gießform 1 platziert werden kann. Der Metallkern 16 kann vier Bohrungen 56 aufweisen, in die die Halterstifte 18 eingesteckt werden. Die Haltestifte 18 können an ihren freien Enden in den Kavitäten 38 der Gießform 1 platziert werden, so dass der Metallkern 16 von den Innenwänden der Gießform beabstandet ist (siehe Figuren 1, 4 bis 11 und 13 bis 15).

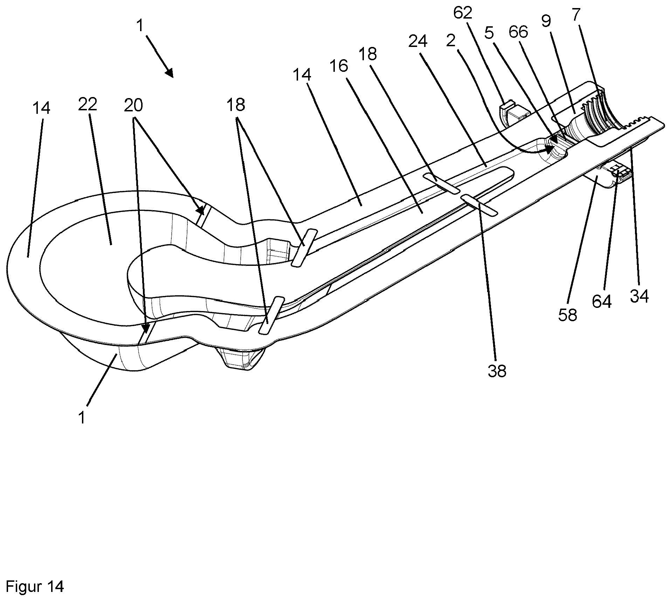

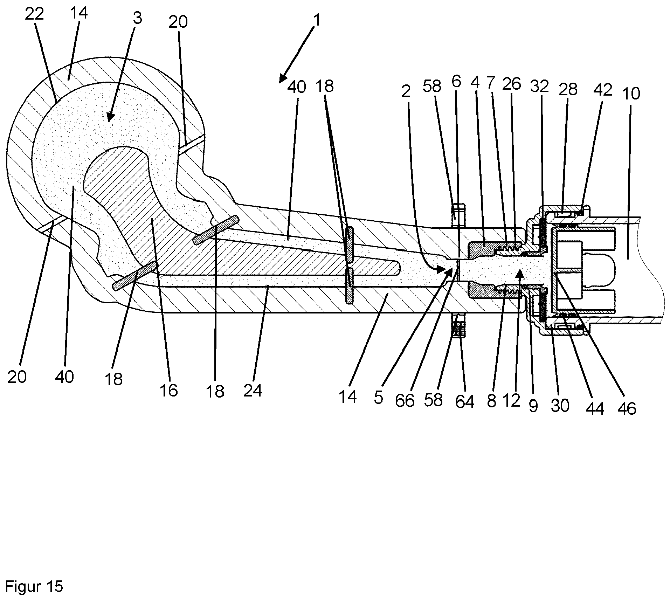

Gemäß einer weiteren alternativen Ausführung, die in den Figuren 13 bis 15 gezeigt ist, kann auch eine zweiteilige Klemmeinrichtung 58 in Form einer Verschlussklemme verwendet werden beziehungsweise Teil der erfindungsgemäßen Vorrichtung sein. Die Klemmeinreichung 58 kann eine Verschlusskante 60 und bevorzugt zwei gegenüberliegende zusätzliche Verschlusskanten aufweisen, mit denen die Leitung 5 in dem verformbaren Bereich 6 derart klemmbar ist, dass der Leitungsquerschnitt sich zumindest so weit verringert, dass der Knochenzementteig 40 nicht mehr hindurchfließen kann. Die beiden Teile der Klemmeinrichtung 58 können über eine Achse 64 miteinander verbunden sein, so dass die Teile um die Achse 64 gegeneinander geschwenkt werden können. Die Klemmeinrichtung 58 bildet dann eine Klammer. Um die Klemmeinrichtung 58 im geschlossenen Zustand halten zu können, kann eine Rastvorrichtung 62 vorgesehen sein, mit der die Klemmeinrichtung 58 im geschlossenen Zustand gehalten werden kann. Auf diese Weise kann der verformbare Bereich 6 der Leitung mit der Klemmeinrichtung 58 abgeklemmt und damit für den Knochenzementteig 40 verschlossen werden. Dadurch erfolgt ein Klemmen der Leitung 5 in dem verformbaren Bereich 6 und es entsteht ein verformter Bereich 66 der Leitung 5, der in den Figuren 14 und 15 gezeigt ist. Der verformte Bereich 66 bildet einen geklemmten Bereich der Leitung 5, durch die der Knochenzementteig 40 nicht hindurchfließen kann. Die Teile der Klemmeinrichtung 58 können aus Kunststoff gefertigt werden, beispielsweise aus thermoplastischem Kunststoff.

Weitere alternative mögliche Klemmeinrichtungen sind vorstellbar, wie beispielsweise eine Schraubklemme oder eine Klammer mit einem Federelement aus Metall.

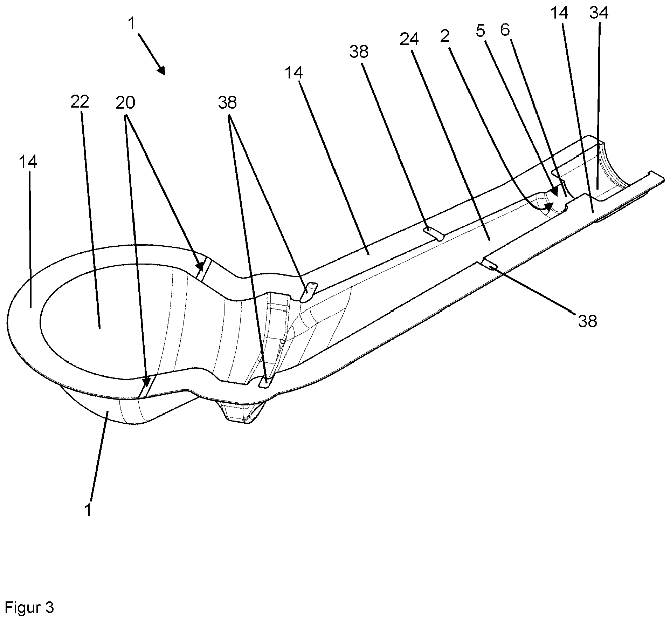

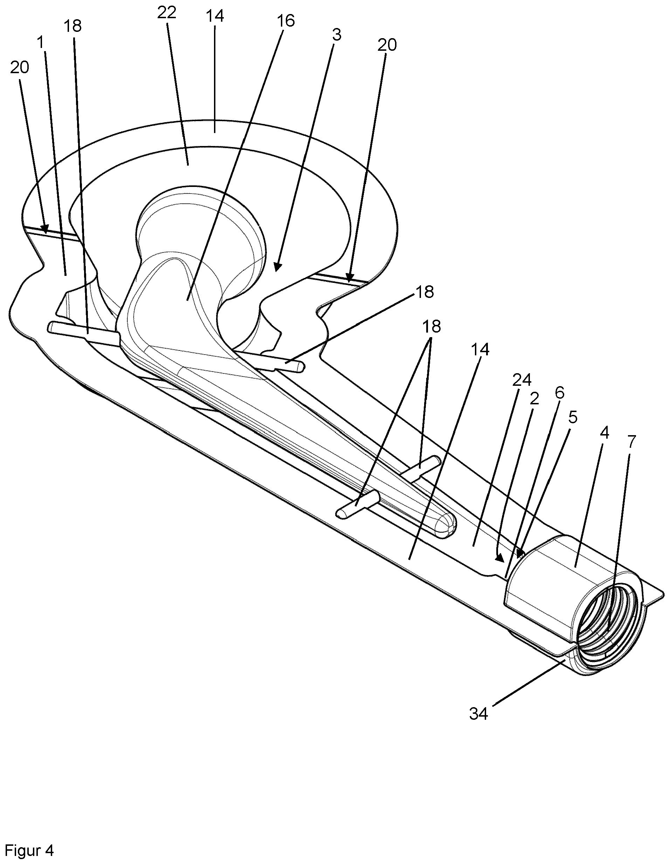

Der Ablauf eines erfindungsgemäßen Verfahrens ist in den Figuren 2 bis 11 und 16 oder 2 bis 7 und 13 bis 16 anhand der erfindungsgemäßen Vorrichtung dargestellt. Die Gießform nach Figur 2 wird bereitgestellt und geöffnet, so wie das in Figur 3 gezeigt ist. Dann kann der Metallkern 16 mit den Haltestiften 18 (siehe Figur 12) in der Gießform 1 positioniert werden. Hierzu können die Haltestifte 18 an einem Ende zwischen den beiden Teilen der Gießform 1 in den durch die Formen 36 gebildeten Kavitäten 38 angeordnet und gehalten und mit dem anderen Ende in den passenden Bohrungen 56 im Metallkern 16 angeordnet werden (siehe Figur 4). Die Gießform 1 kann dann geschlossen werden.

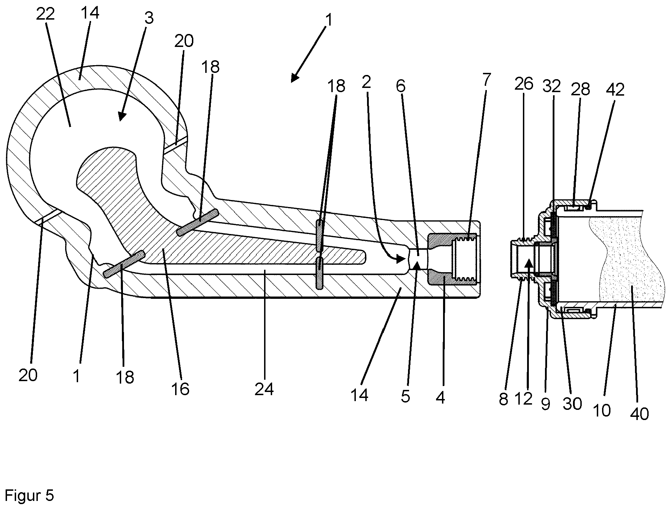

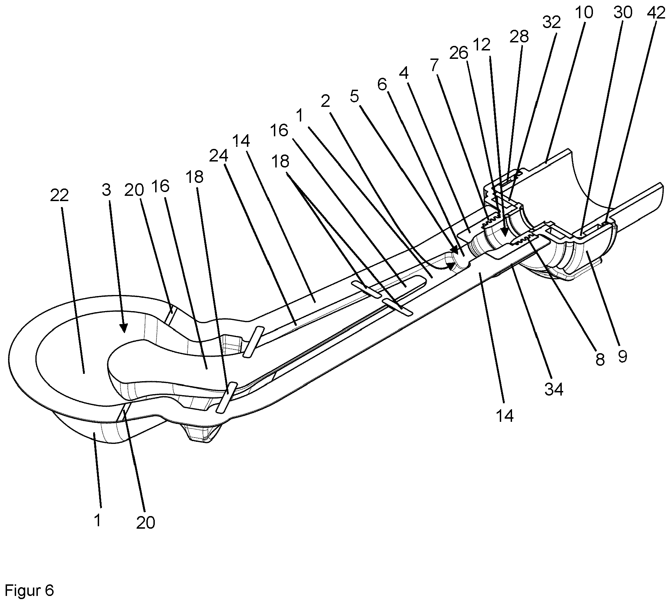

Ein Knochenzementteig 40 kann in der Knochenzementkartusche 10 unter Vakuum gemischt werden (siehe Figur 5). Anschließend kann die Knochenzementkartusche 10 mit dem Adapterelement 9 in den Anschluss 4 geschraubt werden (siehe Figur 6).

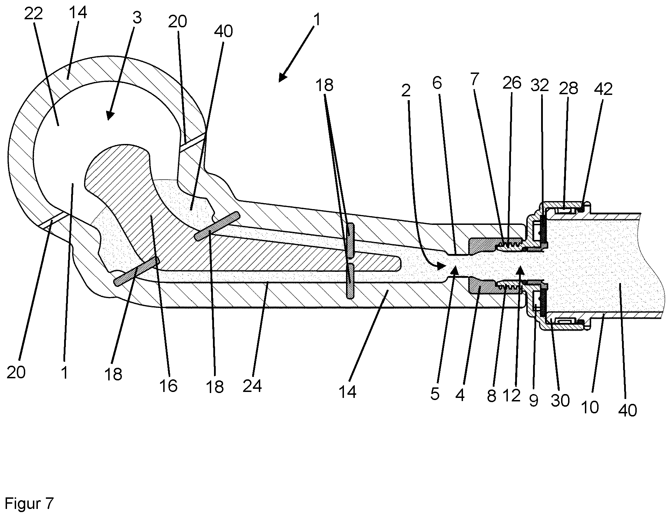

Anschließend wird durch Vortreiben des Kolbens 46 der Knochenzementteig 40 aus der Knochenzementkartusche 10 durch den Anschluss 4 und die Leitung 5 in die Gießform 1 gepresst. Diese Situation ist in Figur 7 gezeigt. Bei Einpressen des Knochenzementteigs 40 wird Luft aus dem Innenraum 3 der Gießform 1 durch die Entlüftungsöffnungen 20 herausgedrückt. Der Knochenzementteig 40 kann dabei den Metallkern 16 allseitig umfließen. Irgendwann ist der Innenraum 3 der Gießform 1 mit dem Knochenzementteig 40 gefüllt (siehe Figuren 8, 9 und 13). Dann kann die Leitung 5 durch Knicken der Leitung 5 im verformbaren Bereich 6 oder durch Klemmen der Leitung im verformbaren Bereich mit einer der Klemmeinrichtungen 48, 58 für den Knochenzementteig 40 undurchlässig verschlossen werden. Diese Situation ist in den Figuren 10 und 11 sowie 14 und 15 gezeigt.

Dabei kann der in der Gießform 1 enthaltene Knochenzementteig 40 nicht wieder ausfließen, da die Leitung 5 durch das Knicken oder Klemmen für den Knochenzementteig 40 undurchlässig geworden ist.

Durch das Klemmen mit der Klemmeinrichtung 48, 58 oder durch das Knicken wird der Knochenzementteig 40 in der Leitung 5 abgetrennt. Die Knochenzementkartusche 10 kann abgeschraubt und entfernt werden. Eventuell verbliebene dünne Verbindungen reißen oder brechen ohne weiteres ab.

In diesem Zustand kann der Knochenzementteig 40 in der Gießform 1 ausgehärtet werden. Anschließend wird der so geformte Spacer 70 aus der Gießform 1 entnommen. Die vorspringenden Haltestifte 18 können abgeschnitten werden. Die abgetrennten Haltestifte 76 sind dann bündig mit der Oberfläche des Spacers 70. Diese Situation ist in Figur 16 gezeigt. Ebenso kann ein Anguss im Bereich des distalen Endes des Schafts abgeschnitten und entfernt werden. Auch können Spitzen, die durch die Entlüftungsöffnungen 20 verursacht werden, entfernt werden. Die Oberfläche des Spacers kann poliert werden und/oder beschichtet werden, beispielsweise mit Antibiotika.

Anstatt einer Gießform 1 zum Formen eines Hüftgelenkspacers kann ohne weiteres auch eine Gießform zum Formen eines anderen Spacers verwendet werden.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Gießform
- 2: Einfüllöffnung
- 3: Innenraum der Gießform
- 4: Anschluss
- 5: Leitung
- 6: Verformbarer Bereich der Leitung
- 7: Gewinde
- 8: Gegengewinde
- 9: Adapterelement
- 10: Knochenzementkartusche
- 12: Austragsöffnung
- 14: Flansch
- 16: Metallkern
- 18: Haltestift
- 20: Entlüftungsöffnung
- 22: Gelenkkopf-Formteil
- 24: Schaft-Formteil
- 26: Stutzen
- 28: Rastmittel
- 30: Gegenrastung
- 32: Porenscheibe
- 34: Form für Anschlussbefestigung
- 36: Form für Kavitäten
- 38: Kavität
- 40: Knochenzementteig
- 42: Dichtung
- 44: Dichtung
- 46: Kolben
- 48: Klemmeinrichtung
- 50: Einschnitt
- 52: Schlitz
- 54: Verformter Bereich der Leitung
- 56: Bohrung
- 58: Klemmeinrichtung
- 60: Verschlusskante
- 62: Rastvorrichtung
- 64: Achse
- 66: Verformter Bereich der Leitung
- 70: Spacer
- 72: Kopf
- 74: Schaft
- 76: Abgetrennter Haltestift

## Patentansprüche

1. Vorrichtung zum Herstellen eines Spacers (70) durch Aushärten von Knochenzementteig (40), wobei der Spacer (70) dazu vorgesehen ist, im medizinischen Bereich ein Gelenk oder einen Teil eines Gelenks umfassend eine artikulierende Oberfläche des Gelenks temporär zu ersetzen, insbesondere ein Hüftgelenk oder ein Schultergelenk temporär zu ersetzen, die Vorrichtung aufweisend eine Gießform (1) mit einem Innenraum (3) zum Formen des Spacers (70) aus Knochenzementteig (40) mit einer Einfüllöffnung (2) zum Einfüllen eines Knochenzementteigs (40),
einen Anschluss (4) zum flüssigkeitsdichten Anschließen einer Knochenzementkartusche (10),
eine Leitung (5) zum Einleiten von Knochenzementteig (40), wobei die Leitung (5) den Anschluss (4) mit der Einfüllöffnung (2) für den Knochenzementteig (40) durchlässig verbindet und die Leitung (5) mit der Einfüllöffnung (2) in den Innenraum (3) der Gießform (1) mündet, wobei die Leitung (5) einen verformbaren Bereich (6) aufweist, der örtlich neben der Einfüllöffnung (2) angeordnet ist,
eine Sollknickstelle im verformbaren Bereich (6) der Leitung (5), wobei der innere Leitungsquerschnitt der Leitung (5) oder die Querschnittsfläche der Leitung (5) durch Knicken der Leitung (5) an der Sollknickstelle der Leitung (5) in dem verformbaren Bereich (6) um mindestens 90% reduzierbar ist, so dass die Leitung (5) in dem so entstandenen Knick für den Knochenzementteig (40) undurchlässig ist, und/oder
eine Klemmeinrichtung (48, 58) zum Klemmen der Leitung (5) im verformbaren Bereich (6), mit der der innere Leitungsquerschnitt der Leitung (5) oder die Querschnittsfläche der Leitung (5) in dem verformbaren Bereich (6) durch Klemmen der Leitung (5) in dem verformbaren Bereich (6) um mindestens 90% reduzierbar ist, so dass die Leitung (5) in dem so geklemmten Zustand in dem geklemmten Bereich für den Knochenzementteig (40) undurchlässig ist.

2. Vorrichtung nach Anspruch 1 **dadurch gekennzeichnet, dass**
der Innenraum (3) der Gießform (1) durch das Knicken oder das Klemmen für den Knochenzementteig (40) nach außen verschließbar ist, so dass der Knochenzementteig (40) nicht aus der Gießform (1) herausfließen kann, wenn die Leitung (5) in dem so geknickten oder in dem so geklemmten Zustand ist, und/oder der verformbare Bereich (6) angrenzend zur Einfüllöffnung (2) angeordnet ist oder der verformbare Bereich (6) der Leitung (5) in einem Abstand von nicht mehr als 10 mm zur Einfüllöffnung (2) beginnt, bevorzugt in einem Abstand von nicht mehr als 2 mm zur Einfüllöffnung (2) beginnt.

3. Vorrichtung nach einem der vorangehenden Ansprüche, **dadurch gekennzeichnet, dass**
die Leitung (5) in dem verformbaren Bereich (6) durch manuelle Krafteinwirkung beim Knicken oder beim Klemmen mit der Klemmeinrichtung (48, 58) derart verformbar ist, dass der innere Leitungsquerschnitt der Leitung (5) oder die Querschnittsfläche der Leitung (5) durch Knicken oder Klemmen der Leitung (5) an der Sollknickstelle der Leitung (5) in dem verformbaren Bereich (6) um mindestens 95% reduzierbar ist, bevorzugt um mindestens 99% reduzierbar ist.

4. Vorrichtung nach einem der vorangehenden Ansprüche, **dadurch gekennzeichnet, dass**
die Leitung (5) zumindest im verformbaren Bereich (6) durch die Gießform (1) gebildet ist, vorzugsweise die gesamte Leitung (5) durch die Gießform (1) gebildet ist, und/oder der Anschluss (4) an einer Seite der Gießform (1) innerhalb der Gießform (1) angeordnet und mit der Gießform (1) verbunden ist, wobei der Anschluss (4) von außerhalb der Gießform (1) zugänglich ist, wobei bevorzugt der Anschluss (4) flüssigkeitsdicht mit der Gießform (1) verbunden ist und den einzigen Zugang für den Knochenzementteig (40) in die Gießform (1) bereitstellt.

5. Vorrichtung nach einem der vorangehenden Ansprüche, **dadurch gekennzeichnet, dass**
die Vorrichtung eine Knochenzementkartusche (10) zum Mischen von Knochenzement-Ausgangskomponenten und zum Austragen von gemischtem Knochenzementteig (40) aus der Knochenzementkartusche (10) heraus aufweist, bevorzugt eine Knochenzementkartusche (10) zum Mischen von Polymethylmethacrylat-Knochenzement-Ausgangskomponenten und zum Austragen von gemischtem Polymethylmethacrylat-Knochenzementteig (40) aus der Knochenzementkartusche (10) heraus aufweist, wobei besonders bevorzugt die Knochenzementkartusche (10) die Knochenzement-Ausgangskomponenten zur Herstellung des Knochenzements in voneinander getrennten Kompartimenten beinhaltet, wobei
die Vorrichtung ein Adapterelement (9) aufweist, das mit der Knochenzementkartusche (10) verbunden ist oder verbindbar ist, wobei das Adapterelement (9) lösbar und formschlüssig mit dem Anschluss (4) verbunden oder verbindbar ist, so dass ein Innenraum (3) der Knochenzementkartusche (10) über das Adapterelement (9) für Knochenzementteig (40) durchlässig mit der Leitung (5) verbunden oder verbindbar ist, wobei vorzugsweise das Adapterelement (9) mit dem Anschluss (4) formschlüssig, lösbar und flüssigkeitsdicht verbindbar oder verbunden ist und/oder das Adapterelement (9) einen Kartuschenkopf der Knochenzementkartusche (10) bildet.

6. Vorrichtung nach einem der vorangehenden Ansprüche, **dadurch gekennzeichnet, dass**
die Gießform (1) und/oder die Leitung (5) aus einer Kunststofffolie besteht oder bestehen oder im Wesentlichen aus einer Kunststofffolie besteht oder bestehen oder die Gießform (1) und/oder die Leitung (5) aus zwei oder mehreren Kunststofffolien aufgebaut ist oder sind, wobei die zwei oder mehreren Kunststofffolien miteinander verschweißt oder verklebt sind, und wobei bevorzugt die Gießform (1) und/oder die Leitung (5) aus PETG-Folie und/oder Polyamid-Folie und/oder PE-Folie gefertigt ist oder sind.

7. Vorrichtung nach einem der vorangehenden Ansprüche, **dadurch gekennzeichnet, dass**
die Einfüllöffnung (2) an einem Teil der Gießform (1) angeordnet ist, der den Schaft (74) des Spacers (70) formt, vorzugsweise an einem Teil der Gießform (1) angeordnet ist, der das distale Ende des Schafts (74) des Spacers (70) formt, und/oder
die Gießform (1) und/oder die Leitung (5) zumindest im verformbaren Bereich (6) elastisch und/oder plastisch verformbar ist oder sind, wobei bevorzugt die Gießform (1) und/oder die Leitung (5) zumindest im verformbaren Bereich (6) plastisch verformbar ist.

8. Vorrichtung nach einem der vorangehenden Ansprüche, **dadurch gekennzeichnet, dass**
der Anschluss (4) zum flüssigkeitsdichten Verbinden einer Knochenzementkartusche (10) ein Gewinde (7) aufweist, insbesondere ein Innengewinde oder ein Außengewinde aufweist, wobei vorzugsweise ein Adapterelement (9) der Knochenzementkartusche (10) oder an der Knochenzementkartusche (10) ein zu dem Gewinde (7) passendes Gegengewinde (8) aufweist, und/oder
der Anschluss (4) aus thermoplastischem Kunststoff besteht, wobei vorzugsweise der Anschluss (4) mit der Leitung (5) und/oder der Gießform (1) verklebt oder verschweißt ist.

9. Vorrichtung nach einem der vorangehenden Ansprüche, **dadurch gekennzeichnet, dass**
die Klemmeinrichtung (48) eine Platte mit einem sich zu einem Inneren der Platte hin verjüngenden Einschnitt (50) ist, wobei der Einschnitt (50) in einen Schlitz (52) mündet, wobei die Breite des Schlitzes (52) kleiner ist als die Dicke der Leitung (5) in dem verformbaren Bereich (6) und durch Einführen des verformbaren Bereichs (6) in den Schlitz (52) das Klemmen der Leitung (5) in dem verformbaren Bereich (6) erfolgt, wobei die Platte bevorzugt einteilig ist, oder
die Klemmeinrichtung (58) zweiteilig oder mehrteilig ist und zumindest zwei der Teile gegeneinander beweglich sind, um die Leitung (5) in dem verformbaren Bereich (6) zu Klemmen und/oder zu Knicken, wobei bevorzugt die Klemmeinrichtung (58) eine Schraubklemme oder eine Verschlussklemme mit einer Rastvorrichtung ist.

10. Vorrichtung nach einem der vorangehenden Ansprüche, **dadurch gekennzeichnet, dass**
die Gießform (1) ausgehend von dem Innenraum (3) der Gießform (1) mindestens drei oder vier Kavitäten (38) zur Aufnahme von Haltestiften (18) aufweist, wobei vorzugsweise die Gießform (1) zweiteilig ist und die Kavitäten (38) in Rändern oder in Flanschen (14) zumindest eines Teils der zweiteiligen Gießform (1) angeordnet sind, und
die Vorrichtung einen Metallkern (16) aufweist, der im Innenraum (3) der Gießform (1) anzuordnen ist, wobei bevorzugt der Metallkern (16) Bohrungen (56) zur Aufnahme von Haltestiften (18) besitzt, wobei besonders bevorzugt die Bohrungen (56) nicht in einem Gelenkkopf-Formteil (22) der Gießform (1) zum Formen einer Gleitfläche des Spacers (70) angeordnet sind, ganz besonders bevorzugt die Bohrungen (56) in einem Schaft-Formteil (24) der Gießform (1) zum Formen eines Schafts (74) des Spacers (70) angeordnet sind.

11. Vorrichtung nach einem der vorangehenden Ansprüche, **dadurch gekennzeichnet, dass**
in der Gießform (1) zumindest eine Entlüftungsöffnung (20) vorgesehen ist, durch die Luft oder Gas aus dem Innenraum (3) der Gießform (1) entweichen kann, wobei bevorzugt wenigstens eine der zumindest einen Entlüftungsöffnung (20) durch einen Spalt geformt ist, der zwischen zwei Teilen der Gießform (1), insbesondere zwischen zwei Flanschen der Gießform (1), beim Anlegen der Teile aneinander verbleibt.

12. Verfahren zur Herstellung eines Spacers (70), der zum temporären Ersetzen eines Gelenks oder eines Teils eines Gelenks, insbesondere eines Hüftgelenks oder eines Schultergelenks, geeignet ist, der Spacer (70) aufweisend eine artikulierende Oberfläche des Gelenks, das Verfahren aufweisend die folgenden chronologischen Schritte:
A) Bereitstellen einer Gießform (1) mit einem Anschluss (4) zum flüssigkeitsdichten Anschließen einer Knochenzementkartusche (10) und einer Leitung (5), die den Anschluss (4) für den Knochenzementteig (40) durchlässig mit einem Innenraum (3) der Gießform (1) verbindet;
B) Einpressen von Knochenzementteig (40) durch den Anschluss (4) und durch die Leitung (5) hindurch in den Innenraum (3) der Gießform (1), bis der Innenraum (3) der Gießform (1) mit Knochenzementteig (40) gefüllt ist;
C) Knicken oder Klemmen der Leitung (5), während fluider Knochenzementteig (40) in der Leitung (5) enthalten ist, wobei sich der freie Leitungsquerschnitt oder die Querschnittsfläche der Leitung (5) durch das Knicken oder Klemmen um mindestens 90% reduziert;
D) Aushärten des Knochenzementteigs (40) in der Gießform (1); und
E) Entnehmen des so geformten und ausgehärteten Spacers (70) aus der Gießform (1).

13. Verfahren nach Anspruch 12, **dadurch gekennzeichnet, dass**
das Verfahren mit einer Vorrichtung nach einem der Ansprüche 1 bis 11 durchgeführt wird.

14. Verfahren nach einem der Ansprüche 12 oder 13, **dadurch gekennzeichnet, dass**
vor Schritt B) eine Knochenzementkartusche (10) an den Anschluss (4) angeschlossen wird, insbesondere in den Anschluss (4) eingeschraubt wird, und in Schritt B) das Einpressen des Knochenzementteigs (40) aus der Knochenzementkartusche (10) erfolgt, wobei bevorzugt zuvor der Knochenzementteig (40) durch Mischen von Ausgangskomponenten, insbesondere einer Monomerflüssigkeit und einer Knochenzementpulvers, innerhalb der Knochenzementkartusche (10) hergestellt wird und besonders bevorzugt die Knochenzementkartusche (10) nach Schritt C) von dem Anschluss (4) getrennt wird, insbesondere aus dem Anschluss (4) herausgeschraubt wird, wobei
das Einpressen des Knochenzementteigs (40) mit Hilfe einer Auspressvorrichtung erfolgt, wobei bevorzugt die Auspressvorrichtung einen Kolben (46) in der Knochenzementkartusche (10) antreibt, und/oder
in Schritt B) die in der Gießform (1) enthaltene Luft durch zumindest eine Entlüftungsöffnung (20) in die Gießform (1) verdrängt wird, während der Knochenzementteig (40) in die Gießform (1) eingepresst wird, wobei vorzugsweise die zumindest eine Entlüftungsöffnung (20) für den Knochenzementteig (40) undurchlässig ist.

15. Verfahren nach einem der Ansprüche 12 bis 14, **dadurch gekennzeichnet, dass**
vor Schritt B), und vorzugsweise vor Schritt A), ein Metallkern (16) innerhalb der Gießform (1) angeordnet wird, wobei bevorzugt der Metallkern (16) über mehrere Haltestifte (18) von einer Innenwand der Gießform (1) beabstandet wird, wobei besonders bevorzugt die mehreren Haltestifte (18) in Bohrungen (56) im Metallkern (16) und in Kavitäten (38) zur Aufnahme der Haltestifte (18) in der Innenwand der Gießform (1) befestigt werden, und/oder
durch das Knicken oder Klemmen in Schritt C) die Leitung (5) für den Knochenzementteig (40) undurchlässig verschlossen wird, vorzugsweise flüssigkeitsdicht verschlossen wird, und/oder
die Gießform (1) zumindest zwei Teile aufweist und dass nach Schritt D) und vor Schritt E) ein Schritt D2) erfolgt:
D2) Öffnen der Gießform (1) durch Trennen der zumindest zwei Teile der Gießform (1) voneinander.
